(19) Europäisches Patentamt
European Patent Office
Office européen des brevets

(11) **EP 3 581 882 A1**

(12) **EUROPÄISCHE PATENTANMELDUNG**

(43) Veröffentlichungstag:
**18.12.2019 Patentblatt 2019/51**

(51) Int Cl.:
**G01B 11/16** *(2006.01)* **G01N 3/20** *(2006.01)*
**G01N 33/32** *(2006.01)*

(21) Anmeldenummer: **19177904.0**

(22) Anmeldetag: **03.06.2019**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **12.06.2018 AT 504712018**

(71) Anmelder: **Technische Universität Wien**
**1040 Wien (AT)**

(72) Erfinder: **Radoevski, Aleksandar**
**1120 Wien (AT)**

(74) Vertreter: **Patentanwaltskanzlei**
**Matschnig & Forsthuber OG**
**Biberstraße 22**
**Postfach 36**
**1010 Wien (AT)**

(54) **BIEGEBALKEN-DEHNMESSVERFAHREN SOWIE PRÜFAUFBAU ZUR DURCHFÜHRUNG EINES SOLCHEN DEHNMESSVERFAHRENS**

(57) Die Erfindung betrifft ein Dehnmessverfahren zur Bestimmung der Dehnfähigkeit dünner Schichten eines Wandanstrichs (20) oder eines mehrlagigen Wandbeschichtungssystems (25), und umfasst die folgenden Schritte:
- Bereitstellen eines Biegebalkens (1) aus einem saugfähigen Material mit einer Balkenlänge (L);
- Anordnen einer Kerbe (2) auf einer Breitseite (11) in der Längenmitte (L/2) des Biegebalkens (1);
wahlweise
- Auftragen eines Wandanstrichs (20) an der der gekerbten Breitseite (11) gegenüberliegenden glatten Breitseite (12);
oder
- Auftragen eines mehrlagigen Wandbeschichtungssystems (25) an der gekerbten Breitseite (11) des Biegebalkens (1);

sowie weiters
- Anordnen von Messpunkten (M) am aufgetragenen Wandanstrich (20) oder Wandbeschichtungssystem (25);
- Aufzeichnen zumindest einer Ausgangslänge ($L_0$) zwischen Messpunkten (M) am unverformten Biegebalken (1);
- Durchführen eines Drei-Punkt-Biegeversuchs mit einer Prüfvorrichtung;
- Aufzeichnen der Biegedeformation des Biegebalkens (1) mit einer Industriekamera (7);
- Beendigen des Drei-Punkt-Biegeversuchs, sobald eine Rissbildung mit Haarrissen wird;
- Ermitteln der Längenänderung $\Delta_L$ des am Biegebalken (1) aufgetragenen Wandanstrichs (20) oder Wandbeschichtungssystems (25).

**Fig. 1**

EP 3 581 882 A1

**Beschreibung**

Die vorliegende Erfindung betrifft ein Dehnmessverfahren zur Bestimmung der Dehnfähigkeit dünner Schichten eines Wandanstrichs oder eines mehrlagigen Wandbeschichtungssystems. Ebenso werden im Rahmen der Erfindung auch Varianten eines Prüfaufbaus zur Durchführung des erfindungsgemäßen Dehnmessverfahrens angegeben, mit welchem Prüfaufbau die Dehnfähigkeit dünner Schichten eines Wandanstrichs oder eines mehrlagigen Wandbeschichtungssystems bestimmt werden kann.

Derzeit gibt es nach Wissen der Anmelderin zumindest in Europa keine normierte Messmethode, die für die Bestimmung der Dehnfähigkeit von Wandanstrichen bzw. von Innenraumbeschichtungen oder die Bestimmung der Dehnfähigkeit sowie des Rissüberbrückungsvermögens von mehrlagigen Wandbeschichtungssystemen für Gebäuderäume geeignet wäre.

Unter dem Begriff einer Innenraumbeschichtung wird im Folgenden ein Wand- oder Deckenanstrich verstanden, der für die Anwendung im Innenraumbereich, gegebenenfalls auch im Außenbereich im Hochbau bestimmt ist. Beispielsweise werden darunter Dispersionsanstriche, Latexanstriche, Silikat-Anstriche oder Kalk-Anstriche verstanden. Derartige Wand- oder Deckenanstriche werden üblicherweise auf weitestgehend rissfreie Gebäudeflächen, beispielsweise Innenraumwände oder Innenraumdecken, aufgetragen.

Unter dem Begriff eines mehrlagigen Wandbeschichtungssystems wird im Weiteren ein System verstanden, welches zur Beschichtung von Gebäudeflächen, welche Risse oder Sprünge aufweisen, verwendet wird. Dazu werden üblicherweise zuerst mit einer Spachtelmasse Risse und Sprünge in der zu beschichtenden Innenraumwand oder Innenraumdecke überspachtelt bzw. überdeckt. Gegebenenfalls kann auch eine Armierungslage, die zweckmäßig beispielsweise als Glasfaservlies ausgeführt ist, mit der Spachtelmasse mit aufgebracht werden, um Mauersprünge bzw. Mauerrisse zu überdecken. Nach dem Aushärten der Spachtelmasse, die erforderlichenfalls noch nachbearbeitet bzw. geschliffen wird, kann dann die rissfreie Gebäudewand oder Gebäudedecke mit einem geeigneten Wand- bzw. Deckenanstrich gestrichen werden. Das mehrlagige Wandbeschichtungssystem umfasst daher jedenfalls eine Spachtelmassenschicht, die abschnittsweise im Bereich von Mauerunebenheiten aufgebracht ist, sowie darüber einen Wandanstrich. Gegebenenfalls kann bei einem solchen Wandbeschichtungssystem auch noch eine zusätzliche Armierungslage vorhanden sein.

Der Einfachheit halber wird im Folgenden kurz von einem Wandanstrich gesprochen, sofern eine Wand- oder Deckenbeschichtung mit einer Anstrichfarbe gemeint ist. Auch witterungsfeste Außenanstriche, die als Fassadenfarben geeignet sind und bei Arbeiten im Außenbereich von Gebäuden zum Einsatz kommen, fallen definitionsgemäß unter den hier verwendeten Begriff des Wandanstrichs.

Überdies wird im Weiteren der Begriff eines Wandbeschichtungssystems verwendet, sobald ein mehrlagiges Beschichtungssystem für die Wand- oder Deckenbeschichtung gemeint ist, welches zumindest einen Abschnitt einer Spachtelmassenschicht samt einem darüber aufgetragenen Wandanstrich betrifft, wobei gegebenenfalls noch eine zusätzliche Armierungslage eine weitere Lage des Wandbeschichtungssystems bilden kann. Der weiterhin verwendete Begriff des Wandbeschichtungssystems umfasst definitionsgemäß sowohl mehrlagige Beschichtungssysteme für die Innenwandbeschichtung, als auch mehrlagige Beschichtungssysteme, die witterungsbeständig sind und für den Einsatz in Außenbereichen beispielsweise von Bauwerksfassaden oder bei Stahlbetonkonstruktionen geeignet sind.

Es existieren lediglich genormte Prüfmethoden zur Bestimmung des Rissüberbrückungsvermögens von Beschichtungen für mineralische Substrate und Beton im Außenbereich (DIN EN 1062-7) sowie für kunststoffmodifizierte Bitumendickbeschichtungen zur Bauwerksabdichtung (DIN EN 15812). Diese Prüfmethoden sind allerdings zur Beurteilung der Dehnfähigkeit sowie des Rissüberbrückungsvermögens von Wandanstrichen oder aber von mehrlagigen Wandbeschichtungssystemen nicht geeignet, da die zu testenden Beschichtungsmaterialien den Belastungen durch die für die vorgenannten Prüfungen etablierten Messvorrichtungen nicht standhalten. Außerdem würden diese ungeeigneten Prüfmethoden bei der Prüfung solcher Wandanstriche oder mehrlagiger Wandbeschichtungssysteme keine vergleichbaren, genauen Messwerte hinsichtlich der Dehnfähigkeit und des Rissüberbrückungsvermögens liefern.

Aktuell werden diese Parameter für Wandanstriche oder mehrlagige Wandbeschichtungssysteme bloß anhand von Erfahrungswerten und Angaben von Verarbeitern wie beispielsweise Malermeistern abgeschätzt.

Aus dem Stand der Technik ist aus dem Dokument DE 19802716 C2 ein Verfahren zur Bestimmung mechanischer Eigenschaften dünner Schichten bekannt geworden, bei dem ein Drei-Punkt-Biegeversuch angewandt wird, um die Streckgrenze, die Zugfestigkeit, die Bruchzähigkeit und den E-Modul von dünnen Schichten, die auf einem metallischen Probekörper aus hochfestem Stahl als Trägermaterial aufgebracht werden. Diese Methode ist jedoch für die Prüfung von dünnen Schichten, wie diese beispielsweise im Maschinenbau Einsatz finden, entwickelt und unter anderem aufgrund des für Wandanstriche oder Wandbeschichtungssysteme völlig ungeeigneten metallischen Trägermaterials als Prüfmethode im Bauwesen nicht geeignet. Überdies verbleibt dort eine Kerbe, die zur Schwächung bzw. als Rissstarter für einen Ermüdungsanriss im Trägermaterial vorgesehen ist, während der Beschichtungsprüfung unverfüllt.

Weiters ist aus dem Stand der Technik aus der Druckschrift DD 266 175 A1 ein Prüfkörper zur Bestimmung des Rissüberbrückungsverfahrens von Anstrichsystemen auf silikatischen Untergründen bekannt geworden. Dabei wird ein Kriechprüfstand mit einem prismatischen Prüfkörper verwendet, der aus einem silikatischen Baustoff besteht und der mit einem modifizierten Betonstahlstab mit reduziertem Durchmesser in der Mitte bewehrt ist. Dieser Prüfkörper wird einem Zugversuch unterzogen, sodass Risse an seiner Oberfläche entstehen. Anschließend wird der Prüfkörper mit einer Dispersionsfarbe beschichtet und nach deren Austrocknung einer weiteren langfristigen Zugprüfung ausgesetzt. Das Rissüberbrückungsverhalten des Anstrichs mit Dispersionsfarbe sowie die Bestimmung der Rissöffnungsverläufe erfolgt dabei anhand der Dehnung des Betonstahlstabes. Eine Dehnfähigkeitsmessung ist mit diesem Kriechprüfstand jedoch nicht möglich. Überdies erfolgt die Dehnungsmessung der Betonstahlbewehrung rein optisch ohne jegliche Messdatenerfassung.

Es besteht somit seit Langem das dringende Bedürfnis, eine Prüfmethode zu entwickeln, die genaue und zuverlässig reproduzierbare Messwerte zur Dehnfähigkeit und zum Rissüberbrückungsvermögen von Wandanstrichen bzw. Innenraumbeschichtungen oder Außenanstrichen oder von mehrlagigen Wandbeschichtungssystemen, die zur Innenraumsanierung oder zur Sanierung von Rissen und Sprüngen an Außenfassaden geeignet sind, liefert. Eine solche Prüfmethode ist weiters von großem wirtschaftlichen Interesse, da anhand von vergleichbaren, reproduzierbaren Messwerten hinsichtlich der Dehnfähigkeit und des Rissüberbrückungsvermögens hinkünftig die Auswahl geeigneter Wandanstriche oder Wandbeschichtungssysteme beispielsweise für die Altbausanierung erleichtert werden soll und dadurch auch Sanierungskosten bei der Innenraumsanierung von Gebäuden eingespart werden sollen.

Die vorliegende Erfindung stellt sich daher die Aufgabe, die aus dem Stand der Technik bekannten Nachteile zu vermeiden, und ein Dehnmessverfahren anzugeben, welches zur Bestimmung der Dehnfähigkeit und des Rissüberbrückungsvermögens von Wandanstrichen oder Wandbeschichtungssystemen geeignet ist.

Erfindungsgemäß umfasst ein Dehnmessverfahren zur Bestimmung der Dehnfähigkeit dünner Schichten eines Wandanstrichs oder eines mehrlagigen Wandbeschichtungssystems die folgenden Schritte:

-a- Bereitstellen eines Biegebalkens aus einem saugfähigen Material, wobei der Biegebalken eine Balkenlänge sowie einen rechteckigen Querschnitt mit einer Balkenbreite sowie mit einer Balkendicke aufweist;

-b- Anordnen einer Kerbe auf einer Breitseite des Biegebalkens in der Längenmitte seiner Balkenlänge sowie quer zur Längsrichtung des Biegebalkens, wobei die Kerbe eine Kerbentiefe sowie eine Kerbenbreite aufweist und über die gesamte Balkenbreite verläuft;

wahlweise - nach einer ersten Prüfanordnung -

-c1- Auftragen eines Wandanstrichs entlang eines Abschnitts mit einer Probenschichtlänge an der der gekerbten Breitseite gegenüberliegenden glatten Breitseite des Biegebalkens beiderseits der Längenmitte seiner Balkenlänge;

oder - alternativ nach einer zweiten Prüfanordnung -

-c2- Auftragen eines mehrlagigen Wandbeschichtungssystems entlang eines Abschnitts mit einer Probenschichtlänge an der gekerbten Breitseite des Biegebalkens beiderseits der Längenmitte seiner Balkenlänge;

sowie weiters

-d- Anordnen von Messpunkten in zumindest einem Probenlängenintervall der Probenschichtlänge des aufgetragenen Wandanstrichs oder des mehrlagigen Wandbeschichtungssystems;

-e- Aufzeichnen zumindest einer Ausgangslänge als Abstandsmaß zwischen Messpunkten des zumindest einen Probenlängenintervalls am unverformten Biegebalken;

-f- Durchführen eines Drei-Punkt-Biegeversuchs mit einer Prüfvorrichtung, wobei der Biegebalken auf zwei seitlichen Auflagern derart positioniert wird, dass die mit dem Wandanstrich oder dem mehrlagigen Wandbeschichtungssystem beschichtete Seite zu den seitlichen Auflagern hin orientiert ist sowie einem in Längenmitte des Biegebalkens mittigen Prüf stempel gegenüberliegt;

-g- Aufzeichnen der Biegedeformation des Biegebalkens während des Drei-Punkt-Biegeversuchs mit einer Industriekamera, die die dem mittigen Prüfstempel gegenüberliegende beschichtete Seite des Biegebalkens erfasst;

-h- Beendigen des Drei-Punkt-Biegeversuchs, sobald in einer Rissbildungszone der beschichteten Seite des Biegebalkens eine Rissbildung mit Haarrissen im Wandanstrich oder im mehrlagigen Wandbeschichtungssystem detektiert wird;

-i- Ermitteln der Längenänderung $\Delta_L$ des am Biegebalken aufgetragenen Wandanstrichs oder Wandbeschichtungssystems als Differenz zumindest einer Zustandslänge bei Rissbildung, welche Zustandslänge als Abstandsmaß zwischen Messpunkten des zumindest einen Probenlängenintervalls bei Rissbildung am deformierten Biegebalken erhalten wird, und der Ausgangslänge des zumindest einen Probenlängenintervalls am unverformten Biegebalken.

Das erfindungsgemäße Dehnmessverfahren zur Bestimmung der Dehnfähigkeit dünner Schichten eines Wandanstrichs oder eines mehrlagigen Wandbeschichtungssystems ist vom Versuchsaufbau her etwa mit dem Drei-Punkt-Biegeversuch nach DIN EN ISO 178 mit dem Titel "Kunststoffe - Bestimmung der Biegeeigenschaften" vergleichbar. Der Aufbau des Drei-Punkt-Biegeversuchs dient bei der Versuchsdurchführung des erfindungsgemäßen Dehnmessverfahrens ebenfalls dazu, eine Biegung eines Biegebalkens zu erzielen, um eine darauf aufgetragene Beschichtung zu testen.

Beim erfindungsgemäßen Dehnmessverfahren wird gemäß Verfahrensschritt -a- zuerst ein Biegebalken aus einem saugfähigen Material bereitgestellt, wobei der Biegebalken eine Balkenlänge sowie einen rechteckigen Querschnitt mit einer Balkenbreite und mit einer Balkendicke aufweist. Das saugfähige Material zur Herstellung des Biegebalkens soll möglichst dieselben bzw. ähnliche Eigenschaften hinsichtlich seiner Saugfähigkeit sowie seiner Biegesteifigkeit haben wie ein mineralischer Untergrund, beispielsweise ein mit Kalkputz oder Zementputz versehenes Mauerwerk eines Innenraums, auf dem üblicherweise Wandanstriche oder mehrlagige Wandbeschichtungssysteme aufgebracht werden. In den Vorversuchen haben sich besonders Trockenbauplatten, insbesondere zementgebundene Spanplatten, als geeignetes Trägermaterial für einen derartigen Biegebalken erwiesen.

Anschließend wird gemäß Verfahrensschritt -b- eine Kerbe auf einer Breitseite des Biegebalkens in der Längenmitte seiner Balkenlänge sowie quer zur Längsrichtung des Biegebalkens angeordnet. Die Kerbe weist eine definierte Kerbentiefe sowie eine definierte Kerbenbreite auf und verläuft über die gesamte Balkenbreite. Die Kerbe führt zu einer bewussten Schwächung des Biegebalkens. Das heißt, während der Biegedeformation wird der Biegebalken im Bereich der Kerbe besonders stark gedehnt.

Mit dem erfindungsgemäßen Dehnmessverfahren können im Weiteren alternativ zwei unterschiedliche Prüfanordnungen realisiert werden. Gemäß Verfahrensschritt -c1- wird nach einer ersten Prüfanordnung ein Wandanstrich entlang eines Abschnitts mit einer Probenschichtlänge an der glatten Breitseite des Biegebalkens, welche der gekerbten Breitseite gegenüberliegt, aufgetragen. Der Auftrag des Wandanstrichs erfolgt dabei auf der glatten Breitseite beiderseits der Längenmitte entlang eines Abschnitts seiner Balkenlänge. Mit dieser ersten Prüfanordnung wird eine Wand- bzw. Deckenbeschichtung simuliert, wie diese beispielsweise auf einer glatten oder bereits zuvor geglätteten Wandfläche eines Innenraums eines Gebäudes als üblicher Wandanstrich aufgebracht wird.

Als Wandanstrich kommen beispielsweise Dispersionsanstriche, Latexanstriche, Silikat-Anstriche oder Kalk-Anstriche in Frage. Überdies hat sich gezeigt, dass mit dem erfindungsgemäßen Dehnmessverfahren auch die Dehnfähigkeit von Außenanstrichen, Fassadenfarben sowie Betonbeschichtungen, die beispielsweise unter dem Begriff "Betonfinish" im Handel erhältlich sind, bestimmt werden können. Solche Außenanstriche werden üblicherweise aufgetragen, damit keine Feuchtigkeit in Putz und Mauerwerk eindringen kann oder um Korrosion auf Metallen zu verhindern. Betonbeschichtungen sind meist polymerhaltige Oberflächenschutzsysteme, die überwiegend auf Oberflächen von Stahlbetonkonstruktionen aufgetragen werden und den Stahlbeton vor äußeren Schädigungsmechanismen während seiner Nutzungsdauer schützen sollen.

Durch die Anordnung der Kerbe auf der dem Wandanstrich gegenüberliegenden Breitseite erfolgt während des Prüfvorgangs eine konzentrierte Dehnungsformation im Bereich der Kerbe in Längenmitte des Biegebalkens. Jener Bereich des Wandanstrichs, der auf der glatten Breitseite gegenüberliegend der Kerbe aufgetragen ist, wird dadurch besonders stark durch Biegedehnung verformt. Die Rissbildung im Wandanstrich auf der glatten Breitseite des Biegebalkens wird somit vorteilhaft auf eine schmale Rissbildungszone eingeschränkt, welche während der Biegedeformation mit einer Industriekamera besonders exakt erfasst werden kann. Wie im Folgenden noch im Detail ausgeführt wird kann mit dieser ersten Prüfanordnung die Dehnfähigkeit $\varepsilon$ des getesteten Wandanstrichs bestimmt werden.

Alternativ zu Verfahrensschritt -c1- kann gemäß einer zweiten Prüfanordnung in einem Verfahrensschritt -c2- ein mehrlagiges Wandbeschichtungssystem getestet werden. Dieses mehrlagige Wandbeschichtungssystem wird entlang eines Abschnitts mit einer Probenschichtlänge an der gekerbten Breitseite des Biegebalkens beiderseits der Längenmitte seiner Balkenlänge aufgetragen. Das mehrlagige Wandbeschichtungssystem wird also - im Gegensatz zu einem zu testenden einfachen Wandanstrich - bewusst auf der gekerbten Breitseite des Biegebalkens aufgetragen. Die Kerbe im Biegebalken simuliert dabei im Dehnmessverfahren einen Riss oder Sprung im Mauerwerk, der vor dem Auftrag eines Wandanstrichs verspachtelt werden muss. Dazu wird eine Spachtelmasse, die für die Anwendung im Bauwesen insbesondere im Trockenbau vorgesehen ist, zur Verfüllung der Kerbe auf der gekerbten Breitseite des Biegebalkens mit einer Schichthöhe bzw. Schichtdicke der Spachtelmasse aufgetragen. Gängige Spachtelmassen zum Verfüllen von Rissen und Sprüngen im Mauerwerk von Innenwänden oder Innendecken eines Gebäudes enthalten üblicherweise Bindemittel wie beispielsweise Gips (Kalziumsulfat-Dihydrat) oder ein Co-Polymer. Ebenso können Spachtelmassen für den Außenbereich getestet werden, die witterungsbeständig sind und bei Fassadenarbeiten zum Verfüllen von Rissen und Sprüngen vor dem Auftrag eines Außenanstrichs eingesetzt werden. Als witterungsbeständige Spachtelmassen für Fassadenarbeiten werden beispielsweise kunstharzvergütete Zementspachtelmassen verwendet.

Überdies kann es erforderlich sein, in die Spachtelmasse eine Armierungslage, beispielsweise einen Fugendeckstreifen aus einem Glasvlies, einzulegen, um die Zugfestigkeit der Spachtelmasse im getrockneten, ausgehärteten Zustand zu erhöhen. Optional ist es also möglich, mit dem Dehnmessverfahren auch Wandbeschichtungssysteme, die mit einer oder mit mehreren Armierungslagen verstärkt sind, zu testen. Nach dem Aushärten der Spachtelmasse, die gegebenenfalls auch mit einer Armierungslage verstärkt ist, und einem optionalen weiteren Bearbeitungsschritt, bei dem die Spachtelmasse noch geglättet oder geschliffen wird, wird abschließend ein Wandanstrich als Deckschicht des Wandbeschichtungssystems auf der Spachtelmasse aufgebracht.

Durch die Anordnung des Wandbeschichtungssystems auf der gekerbten Breitseite erfolgt während des Prüfvorgangs eine konzentrierte Dehnungsdeformation im Bereich der Kerbe in Längenmitte des Biegebalkens. Jener Bereich des Wandbeschichtungssystems, der auf der gekerbten Breitseite im Bereich direkt angrenzend an die Kerbe aufgetragen ist, wird dadurch besonders stark verformt. Die Rissbildung im Wandbeschichtungssystem auf der gekerbten Breitseite des Biegebalkens wird somit vorteilhaft auf eine schmale Rissbildungszone im Bereich der überspachtelten Kerbe eingeschränkt, welche während der Biegedeformation wiederum mit einer Industriekamera besonders exakt erfasst werden kann.

Wie im Folgenden noch im Detail ausgeführt wird, können mit dieser zweiten Prüfanordnung sowohl die Dehnfähigkeit ε des getesteten mehrlagigen Wandbeschichtungssystems, als auch dessen Rissüberbrückungsvermögen bestimmt werden.

Die nachfolgenden weiteren Verfahrensschritte -d- bis -i- werden für die beiden unterschiedlichen Prüfanordnungen jeweils analog durchgeführt. Gemäß Verfahrensschritt -d- werden entsprechende Messpunkte in zumindest einem Probenlängenintervall der Probenschichtlänge des aufgetragenen Wandanstrichs oder des mehrlagigen Wandbeschichtungssystems aufgetragen. Die Intervallabschnitte eines oder mehrerer Probenlängenintervalle werden dabei jeweils symmetrisch von der Längenmitte des Biegebalkens aus in dessen Längsrichtung aufgetragen. Die Längenmitte des Biegebalkens ist aufgrund der dort angeordneten Kerbe auf einer der beiden Breitseiten die neuralgische Schwachstelle des Biegebalkens, bei der während der Biegedeformation auch zuerst die Rissbildungszone auftritt.

Gemäß Verfahrensschritt -e- erfolgt anschließend am noch unverformten Biegebalken eine exakte Erfassung und Aufzeichnung der Ausgangslängen der Probenlängenintervalle als Abstandsmaße zwischen Messpunkten der entsprechenden Probenlängenintervalle.

Im nachfolgenden Verfahrensschritt -f- wird ein Drei-Punkt-Biegeversuch mit einer entsprechenden Prüfvorrichtung durchgeführt, wobei der Biegebalken auf zwei seitlichen Auflagern derart positioniert wird, dass die mit dem Wandanstrich oder dem mehrlagigen Wandbeschichtungssystem beschichtete Seite zu den seitlichen Auflagern hin orientiert ist sowie einem in Längenmitte des Biegebalkens mittigen Prüfstempel gegenüberliegt. Der zu prüfende Biegebalken wird also so in die Prüfvorrichtung eingelegt, dass der mittig des Biegebalkens angeordnete Prüfstempel, der auf der gegenliegenden Seite der beiden Auflager der Prüfvorrichtung positioniert ist, jeweils auf die nicht bestrichene bzw. nicht beschichtete Breitseite des Biegebalkens drückt. Der zu testende Wandanstrich oder das zu testende Wandbeschichtungssystem befindet sich daher auf der dem mittig angeordneten Prüfstempel gegenüberliegenden sowie diesem abgewandten Breitseite des Biegebalkens, welche während der Biegedeformation gedehnt wird.

Zweckmäßig ist die Prüfvorrichtung zur Krafteinleitung während der Biegedeformation mit Auflagern sowie mit einem Prüfstempel ausgestattet, welche Prüfstandsrollen als Rollenlager aufweisen. Damit wird eine Drehmoment- und verschiebungsfreie Lagerung bzw. Biegebelastung des Biegebalkens während der Biegedeformation gewährleistet. Vorteilhaft weisen solche Prüfstandsrollen für die Lagerung an den beiden Auflagern sowie am freien Ende des Prüfstempels jeweils die gleichen Abmessungen auf - also denselben Rollendurchmesser sowie jeweils eine Zylinderlänge, die zumindest der Balkenbreite des Biegebalkens entspricht - und sind zweckmäßig aus demselben Material, beispielsweise aus Metall, vorzugsweise aus Stahl, gefertigt.

Gemäß Verfahrensschritt -g- erfolgt während des Drei-Punkt-Biegeversuchs, also während der Biegedeformation des Biegebalkens, eine Aufzeichnung der Rissbildung im zu testenden Wandanstrich bzw. im zu testenden Wandbeschichtungssystem mit einer Industriekamera. Die Industriekamera erfasst dabei die dem mittigen Prüfstempel gegenüberliegende beschichtete Seite des Biegebalkens.

Zweckmäßig wird dazu die Rissbildung während der Biegedeformation des Biegebalkens mit einer optischen Verformungsanalyse mittels digitaler Bildkorrelation (auf Englisch kurz: DIC, digital image correlation) erfasst. Dazu wird eine Industriekamera, die die Verformung am zu prüfenden Biegebalken während des Biegevorgangs aufzeichnet, und eine entsprechende Mess-Software, die die aufgezeichneten Verformungen und Verschiebungen speichert und auswertet, benötigt. Diese Komponenten - also die Industriekamera samt zugehöriger Mess-Software, ein entsprechender Mess-Computer zur Erfassung, Speicherung und Auswertung der Messdaten sowie die gegebenenfalls benötigte Verkabelung zur Signalverbindung der einzelnen Komponenten - bilden einen sogenannten Videoextensometer. Die Funktionsweise und der Aufbau eines solchen Videoextensometers sind dem Fachmann auf dem Gebiet von Dehnmessverfahren bekannt. Die Bestimmung von Längenverschiebungen bzw. von Dehnungen aufgrund der Biegedeformation des Biegebalkens erfolgt mit dem Videoextensometer anhand von Messpunkten, die am Biegebalken im gewünschten Messbereich der Probenschichtlänge des Wandanstrichs bzw. des Wandbeschichtungssystems aufgebracht werden. Diese Messpunkte werden vorteilhaft so ausgewählt bzw. sind derart gestaltet, dass sie in der Darstellung der Industriekamera als Grauwertmuster bzw. Specklemuster detektiert werden können.

Besonders zweckmäßig werden die Messpunkte dabei in mehreren Probenlängenintervallen beiderseits der Längenmitte des Biegebalkens im Bereich der Probenschichtlänge aufgebracht. Beispielsweise wird standardgemäß ein erstes Probenlängenintervall derart gewählt, dass dessen Intervalllänge einem Zehntel der gesamten Balkenlänge des Biegebalkens entspricht. Ein zweites Probenlängenintervall wird beispielsweise derart gewählt, dass dessen Intervalllänge einem Drittel der gesamten Balkenlänge des Biegebalkens entspricht. Für den Fall der zweiten Prüfanordnung, wenn also ein mehrlagiges Wandbeschichtungssystem auf der gekerbten Breitseite des Biegebalkens aufgetragen wird, wird zweckmäßigerweise noch ein drittes Probenlängenintervall derart mit Messpunkten auf dem Wandbeschichtungssystem aufgebracht, sodass die Messpunkte direkt an die überspachtelte Kerbe angrenzen. mit diesem dritten Probenlängenintervall, dessen Intervalllänge etwa der Summe der Kerbenbreite samt zweimal der halben Messpunktbreite entspricht, kann das Rissüberbrückungsvermögen des Wandbeschichtungssystems bestimmt werden.

Gemäß Verfahrensschritt -h- wird der Drei-Punkt-Biegeversuch beendigt, sobald von der Industriekamera bzw. dem Videoextensometer in einer Rissbildungszone der beschichteten Seite des Biegebalkens eine Rissbildung mit Haarrissen im Wandanstrich oder im mehrlagigen Wandbeschichtungssystem detektiert wird.

Abschließend erfolgt gemäß Verfahrensschritt -i- die Ermittlung der Längenänderung $\Delta_L$ des am Biegebalken aufgetragenen Wandanstrichs oder Wandbeschichtungssystems als Differenz zumindest einer Zustandslänge bei Rissbildung, welche Zustandslänge als Abstandsmaß zwischen Messpunkten des zumindest einen Probenlängenintervalls bei Rissbildung am deformierten Biegebalken erhalten wird, und der Ausgangslänge des zumindest einen Probenlängenintervalls am unverformten Biegebalken. Es erfolgt also ein Vergleich der Zustandslängen eines oder mehrerer Probenlängenintervalle bei bereits erkennbarer Rissbildung nach der Biegedeformation mit den jeweils korrelierenden Probenlängenintervallen vor der Biegedeformation des Biegebalkens.

Vorteilhaft kann mit einem Dehnmessverfahren gemäß der Erfindung die Dehnfähigkeit $\varepsilon$ des getesteten Wandanstrichs oder des mehrlagigen Wandbeschichtungssystems als Verhältnis der Längenänderung $\Delta_L$ des Biegebalkens zur ursprünglichen Ausgangslänge durch Berechnung ermittelt werden, wobei die Längenänderung $\Delta_L$ als Differenz von deformierter Zustandslänge bei Rissbildung zur ursprünglichen Ausgangslänge ausgedrückt wird. Für die Berechnung der maximalen Dehnfähigkeit $\varepsilon$ (in [%]) des getesteten Wandanstrichs oder des mehrlagigen Wandbeschichtungssystems wird zuerst die Längenänderung $\Delta_L$ des Biegebalkens (in [mm]) als Differenz von deformierter Zustandslänge $L_1$ bei Rissbildung zur ursprünglichen Ausgangslänge $L_0$ vor der Biegedeformation nach der folgenden Formel (1) berechnet:

$$\Delta_L = L_1 - L_0 \qquad \text{in } [\text{mm}] \qquad\qquad (1)$$

Gemäß der folgenden Formel (2) wird die Dehnfähigkeit $\varepsilon$ des getesteten Wandanstrichs oder des mehrlagigen Wandbeschichtungssystems als Verhältnis der Längenänderung $\Delta_L$ des Biegebalkens zur ursprünglichen Ausgangslänge $L_0$ ausgedrückt:

$$\varepsilon = (\Delta_L \, / \, L_0) * 100 \qquad \text{in } [\%] \qquad\qquad (2)$$

Vorteilhafterweise kann mit einem erfindungsgemäßen Dehnmessverfahren ebenfalls das Rissüberbrückungsvermögen $L_R$ des getesteten mehrlagigen Wandbeschichtungssystems als Differenz von deformierter Zustandslänge des Probenlängenintervalls bei Rissbildung, welches Probenlängenintervall direkt an die Kerbe angrenzende Messpunkte aufweist, zur ursprünglichen Ausgangslänge dieses Probenlängenintervalls mit direkt an die Kerbe angrenzenden Messpunkten, berechnet werden.

Für die Bestimmung des Rissüberbrückungsvermögens des getesteten mehrlagigen Wandbeschichtungssystems mit der zweiten Prüfanordnung, wenn also ein mehrlagiges Wandbeschichtungssystem auf der gekerbten Breitseite des Biegebalkens aufgetragen wird, wird ein drittes Probenlängenintervall derart mit Messpunkten auf dem Wandbeschichtungssystem aufgebracht, wobei die Messpunkte dieses dritten Probenlängenintervalls direkt an die überspachtelte Kerbe angrenzen. Mit diesem dritten Probenlängenintervall, dessen Intervalllänge etwa der Summe der Kerbenbreite samt dem zweifachen Wert der halben Messpunktbreite der direkt an die Kerbe angrenzenden Messpunkte entspricht, kann das Rissüberbrückungsvermögen $L_R$ (in [mm]) des Wandbeschichtungssystems als Differenz von deformierter Zustandslänge $L_{R3}$ des Probenlängenintervalls bei Rissbildung, welches Probenlängenintervall direkt an die Kerbe angrenzende Messpunkte aufweist, zur ursprünglichen Ausgangslänge $L_{P3}$ dieses Probenlängenintervalls mit direkt an die Kerbe angrenzenden Messpunkten, gemäß der folgenden Formel (3) berechnet werden:

$$L_R = L_{R3} - L_{P3} \qquad \text{in } [\text{mm}] \qquad\qquad (3)$$

Besonders zweckmäßig kann bei einem Dehnmessverfahren gemäß der Erfindung die deformierte Zustandslänge bei Rissbildung von Haarrissen im Wandanstrich oder im mehrlagigen Wandbeschichtungssystem mit einer Rissöffnung von zumindest 0,2 mm bestimmt werden. Haarrisse mit einer Rissöffnung von zumindest 0,2 mm haben den Vorteil, dass diese auch bereits mit freiem Auge erkannt werden können. Überdies hat sich dieses Maß für Rissöffnungen auch bereits in Normen niedergeschlagen. So werden beispielsweise auch gemäß der DIN 18550-1 ("Planung, Zubereitung und Ausführung von Innen- und Außenputzen") Risse erst ab einer Breite von 0,2 mm als optische und/oder technische Mängel bezeichnet. Mit Hilfe der Industriekamera bzw. des Videoextensometers gelingt es, bei der nachträglichen Auswertung der Biegedeformation des Biegebalkens jenen Zeitpunkt der Rissbildung zu ermitteln, bei dem in der Rissbildungszone erstmals entsprechende Haarrisse mit 0,2 mm Rissöffnung aufgetreten sind. Die zu diesem Zeitpunkt während der Biegeverformung dazugehörigen Zustandslängen $L_1$ werden dann, wie zuvor beschrieben, für die weitere Auswertung zur Bestimmung der Dehnfähigkeit bzw. des Rissüberbrückungsvermögens herangezogen.

In einer besonders zweckmäßigen Variante des erfindungsgemäßen Dehnmessverfahrens kann der bereitgestellte Biegebalken eine Balkendicke von 10 mm bis 40 mm, bevorzugt von 20 mm bis 28 mm, besonders bevorzugt von 24 mm, aufweisen. Diese Balkendicken haben sich in Vorversuchen als besonders zweckmäßig für die Durchführung der Biegeprüfung erwiesen. Einerseits sind derartige Biegebalken noch dünn genug, um von einer Person hantiert werden zu können. Andererseits sind derartige Biegebalken ausreichend dick, um an einer Breitseite des Biegebalkens auch eine durchgehende Kerbe quer zur Längsrichtung des Biegebalkens anordnen zu können.

Vorteilhaft kann es sein, wenn bei einem Dehnmessverfahren gemäß der Erfindung der bereitgestellte Biegebalken eine Balkenbreite aufweist, die dem dreifachen Wert der Balkendicke entspricht, sowie eine Balkenlänge aufweist, die dem zwölffachen Wert der Balkendicke entspricht. Besonders zweckmäßig werden beispielsweise Biegebalken verwendet, die eine Balkenlänge von 300 mm, eine Balkenbreite von 75 mm sowie eine Balkendicke von 24 mm aufweisen.

Zweckmäßig kann bei einem erfindungsgemäßen Dehnmessverfahren der bereitgestellte Biegebalken eine Kerbe mit einer Kerbentiefe aufweisen, die von 20 % bis 80 % der Balkendicke, bevorzugt von 40 % bis 60 % der Balkendicke, besonders bevorzugt 50% der Balkendicke, beträgt. Wesentlich ist dabei, dass die gewählte Kerbentiefe bei den beiden Prüfanordnungen nicht die Tragfähigkeit des Biegebalkens beeinträchtigt, sodass dieser bereits vor dem Prüfvorgang aufgrund seines Eigengewichtes versagt oder Biegedeformationen erfährt.

In einer weiteren vorteilhaften Ausführung kann bei einem Dehnmessverfahren gemäß der Erfindung der bereitgestellte Biegebalken eine Kerbe mit einer Kerbenbreite von 2 mm bis 4 mm aufweisen. Zweckmäßig kann eine Kerbe mit dieser Kerbenbreite beispielsweise mit einer Kreissäge am Biegebalken angeordnet werden.

Besonders vorteilhaft kann es sein, wenn bei einem Dehnmessverfahren gemäß der Erfindung ein Biegebalken bereitgestellt wird, welcher aus einer Komposit-Platte enthaltend Holz und Zement hergestellt ist. In Vorversuchen hat sich herausgestellt, dass solche sogenannte Zement-gebundene Spanplatten als Komposit-Materialien besonders gut als Biegebalken geeignet sind, da diese vorteilhaft ein ähnliches Saugverhalten wie ein mineralischer Putz aufweisen und eine ausreichende Festigkeit und eine gute Verarbeitbarkeit besitzen.

Zweckmäßig wird bei einem Dehnmessverfahren gemäß der Erfindung ein Biegebalken bereitgestellt bzw. eingesetzt, der

- von 50 Vol.-% bis 70 Vol.-% an Holz oder Holzspänen,
- von 20 Vol.-% bis 40 Vol.-% an Zement,
- von 7 Vol.-% bis 15 Vol.-% an gebundenem Wasser sowie
- von 0 Vol.-% bis 3 Vol.-% an zusätzlichen Hydratationszusätzen enthält.

Komposit-Platten mit einer derartigen Zusammensetzung, die Zement als hydraulisches Bindemittel und gegebenenfalls noch weitere, zusätzliche Bindemittel als Hydratationszusätze enthalten, bieten den Vorteil, ausreichend saugfähig zu sein zum Auftragen eines Wandanstrichs oder eines mehrlagigen Wandbeschichtungssystems sowie eine ausreichende Biegesteifigkeit aufzuweisen, um sich nicht unerwünschter Weise bereits vor dem Prüfvorgang aufgrund ihres Eigengewichtes zu deformieren.

Die eingangs genannten Aufgaben der Erfindung werden auch mit einem Prüfaufbau zur Durchführung eines erfindungsgemäßen Dehnmessverfahrens gelöst.

Gemäß einer ersten Prüfanordnung umfasst ein erfindungsgemäßer Prüfaufbau zur Durchführung eines Dehnmessverfahrens zur Bestimmung der Dehnfähigkeit dünner Schichten eines Wandanstrichs

- einen Biegebalken sowie eine mit einer Industriekamera sowie mit einer Mess-Software ausgestattete Prüfvorrichtung zur Durchführung eines Drei-Punkt-Biegeversuchs, wobei die Industriekamera dazu ausgebildet ist, die Verformung am zu prüfenden Biegebalken während des Biegevorgangs aufzuzeichnen, und die Mess-Software dazu ausgebildet ist, die aufgezeichneten Verformungen und Verschiebungen zu speichern und auszuwerten; wobei
- der Biegebalken aus einem saugfähigen Material bereitgestellt ist, eine Balkenlänge sowie einen rechteckigen Querschnitt mit einer Balkenbreite und mit einer Balkendicke aufweist, wobei eine Kerbe auf einer Breitseite des Biegebalkens in der Längenmitte seiner Balkenlänge sowie quer zur Längsrichtung des Biegebalkens angeordnet ist, wobei die Kerbe eine Kerbentiefe sowie eine Kerbenbreite aufweist und über die gesamte Balkenbreite verläuft; sowie
- entlang eines Abschnitts mit einer Probenschichtlänge an der der gekerbten Breitseite gegenüberliegenden glatten Breitseite des Biegebalkens beiderseits der Längenmitte seiner Balkenlänge ein Wandanstrich aufgetragen ist; sowie weiters

- Messpunkte in zumindest einem Probenlängenintervall der Probenschichtlänge des aufgetragenen Wandanstrichs angeordnet sind; wobei
- zumindest eine Ausgangslänge als Abstandsmaß zwischen Messpunkten des zumindest einen Probenlängenintervalls am unverformten Biegebalken aufgezeichnet ist; wobei
- der Biegebalken zur Durchführung eines Drei-Punkt-Biegeversuchs auf zwei seitlichen Auflagern der Prüfvorrichtung derart positioniert ist, dass die mit dem Wandanstrich beschichtete Seite zu den seitlichen Auflagern hin orientiert ist sowie einem in Längenmitte des Biegebalkens mittig angeordneten Prüfstempel der Prüfvorrichtung gegenüberliegt; wobei
- die Aufzeichnung der Biegedeformation des Biegebalkens während des Drei-Punkt-Biegeversuchs mit einer Industriekamera erfolgt, welche Industriekamera die dem mittigen Prüfstempel gegenüberliegende beschichtete Seite des Biegebalkens erfasst; sowie
- die Prüfvorrichtung derart konfiguriert ist, dass der Drei-Punkt-Biegeversuch endet, sobald die Industriekamera in einer Rissbildungszone der beschichteten Seite des Biegebalkens eine Rissbildung mit Haarrissen im Wandanstrich detektiert.

In einer alternativen Konfiguration nach einer zweiten Prüfanordnung umfasst ein erfindungsgemäßer Prüfaufbau zur Durchführung eines Dehnmessverfahrens zur Bestimmung der Dehnfähigkeit dünner Schichten eines mehrlagigen Wandbeschichtungssystems

- einen Biegebalken sowie eine mit einer Industriekamera sowie mit einer Mess-Software ausgestattete Prüfvorrichtung zur Durchführung eines Drei-Punkt-Biegeversuchs umfasst, wobei die Industriekamera dazu ausgebildet ist, die Verformung am zu prüfenden Biegebalken während des Biegevorgangs aufzuzeichnen, und die Mess-Software ausgebildet ist, die aufgezeichneten Verformungen und Verschiebungen zu speichern und auszuwerten; wobei
- der Biegebalken aus einem saugfähigen Material bereitgestellt ist, eine Balkenlänge sowie einen rechteckigen Querschnitt mit einer Balkenbreite und mit einer Balkendicke aufweist, wobei eine Kerbe auf einer Breitseite des Biegebalkens in der Längenmitte seiner Balkenlänge sowie quer zur Längsrichtung des Biegebalkens angeordnet ist, wobei die Kerbe eine Kerbentiefe sowie eine Kerbenbreite aufweist und über die gesamte Balkenbreite verläuft; sowie
- entlang eines Abschnitts mit einer Probenschichtlänge an der gekerbten Breitseite des Biegebalkens beiderseits der Längenmitte seiner Balkenlänge ein mehrlagiges Wandbeschichtungssystem aufgetragen ist; sowie weiters
- Messpunkte in zumindest einem Probenlängenintervall der Probenschichtlänge des aufgetragenen Wandanstrichs angeordnet sind; wobei
- zumindest eine Ausgangslänge als Abstandsmaß zwischen Messpunkten des zumindest einen Probenlängenintervalls am unverformten Biegebalken aufgezeichnet ist; wobei
- der Biegebalken zur Durchführung eines Drei-Punkt-Biegeversuchs auf zwei seitlichen Auflagern der Prüfvorrichtung derart positioniert ist, dass die mit dem Wandanstrich beschichtete Seite zu den seitlichen Auflagern hin orientiert ist sowie einem in Längenmitte des Biegebalkens mittig angeordneten Prüfstempel der Prüfvorrichtung gegenüberliegt; wobei
- die Aufzeichnung der Biegedeformation des Biegebalkens während des Drei-Punkt-Biegeversuchs mit einer Industriekamera erfolgt, welche Industriekamera die dem mittigen Prüfstempel gegenüberliegende beschichtete Seite des Biegebalkens erfasst; sowie
- die Prüfvorrichtung derart konfiguriert ist, dass der Drei-Punkt-Biegeversuch endet, sobald die Industriekamera in einer Rissbildungszone der beschichteten Seite des Biegebalkens eine Rissbildung mit Haarrissen im Wandanstrich detektiert.

Wie eingangs bereits festgehalten ist, bilden bestimmte Komponenten der Prüfvorrichtung - also die Industriekamera samt zugehöriger Mess-Software, ein entsprechender Mess-Computer zur Erfassung, Speicherung und Auswertung der Messdaten sowie die gegebenenfalls benötigte Verkabelung zur Signalverbindung der einzelnen Komponenten - einen sogenannten Videoextensometer. Die Funktionsweise und der Aufbau eines solchen Videoextensometers sind dem Fachmann auf dem Gebiet von Dehnmessverfahren bekannt. Die Bestimmung von Längenverschiebungen bzw. von Dehnungen aufgrund der Biegedeformation des Biegebalkens erfolgt mit dem Videoextensometer anhand von Messpunkten, die am Biegebalken im gewünschten Messbereich der Probenschichtlänge des Wandanstrichs bzw. des Wandbeschichtungssystems in einem oder in mehreren Probenlängenintervallen aufgebracht sind. Diese Messpunkte werden vorteilhaft so ausgewählt bzw. sind derart gestaltet, dass sie in der Darstellung der Industriekamera als Grauwertmuster bzw. Specklemuster detektiert werden können.

In einer vorteilhaften Weiterbildung der Erfindung kann bei einem Prüfaufbau nach Beendigung des Drei-Punkt-Biegeversuchs die Mess-Software der Prüfvorrichtung die Längenänderung $\Delta_L$ des am Biegebalken aufgetragenen Wandanstrichs oder Wandbeschichtungssystems als Differenz zumindest einer Zustandslänge bei Rissbildung, welche Zustandslänge dem Abstandsmaß zwischen Messpunkten des zumindest einen Probenlängenintervalls bei Rissbildung am deformierten Biegebalken entspricht, und der Ausgangslänge des zumindest einen Probenlängenintervalls am unverformten Biegebalken durch Berechnung ermitteln.

Mit einem Prüfaufbau gemäß der Erfindung kann solcherart gemäß der ersten Prüfanordnung für einen zu testenden Wandanstrich die durch die Biegedeformation verursachte Längenänderung $\Delta_L$ des am Biegebalken aufgetragenen Wandanstrichs bestimmt werden. Anschließend kann die Dehnfähigkeit $\varepsilon$ des getesteten Wandanstrichs anhand des vorhin dargelegten Berechnungsschemas ermittelt werden.

Mit einem erfindungsgemäßen Prüfaufbau kann ebenso gemäß der zweiten Prüfanordnung für ein zu testendes mehrlagiges Wandbeschichtungssystem die durch die Biegedeformation verursachte Längenänderung $\Delta_L$ des am Biegebalken aufgetragenen Wandbeschichtungssystems bestimmt werden. Anschließend kann auch für das getestete mehrlagige Wandbeschichtungssystem die Dehnfähigkeit $\varepsilon$ anhand des vorhin dargelegten Berechnungsschemas ermittelt werden. Ebenso kann das Rissüberbrückungsvermögen des getesteten mehrlagigen Wandbeschichtungssystems anhand des vorhin dargelegten Berechnungsschemas ermittelt werden.

Weitere Einzelheiten, Merkmale und Vorteile der Erfindung ergeben sich aus der nachfolgenden Erläuterung von in den Zeichnungen schematisch dargestellten Ausführungsbeispielen. In den Zeichnungen zeigen:

- **Fig. 1**  in einer Seitenansicht eine erste Prüfanordnung des Dehnmessverfahrens gemäß der Erfindung mit einem Biegebalken zur Prüfung der Dehnfähigkeit eines Wand- bzw. Deckenanstrichs;
- **Fig. 1A**  eine vergrößerte Detailansicht der in Fig. 1 gezeigten ersten Prüfanordnung in Ausgangslage mit einem unverformten Biegebalken;
- **Fig. 1B**  die in Fig. 1A gezeigte erste Prüfanordnung nach einer Biegedeformation des Biegebalkens;
- **Fig. 2**  in einer Seitenansicht eine zweite Prüfanordnung des Dehnmessverfahrens gemäß der Erfindung mit einem Biegebalken zur Prüfung der Dehnfähigkeit sowie des Rissüberbrückungsvermögens eines mehrlagigen Wand- bzw. Deckenbeschichtungssystems;
- **Fig. 2A**  eine vergrößerte Detailansicht der in Fig. 2 gezeigten zweiten Prüfanordnung in Ausgangslage mit einem unverformten Biegebalken;
- **Fig. 2B**  die in Fig. 2A gezeigte zweite Prüfanordnung nach einer Biegedeformation des Biegebalkens;
- **Fig. 3**  in einer isometrischen Ansicht schräg von vorne einen Biegebalken mit einer Kerbe mittig und quer zur Längsrichtung des Biegebalkens;
- **Fig. 4**  in einer schematischen Seitenansicht den Prüfaufbau eines Biegebalken-Dehnmessverfahrens unter Einsatz einer hochauflösenden Industriekamera;
- **Fig. 5**  in einer Draufsicht die glatte Breitseite eines für die erste Prüfanordnung des erfindungsgemäßen Dehnmessverfahrens vorbereiteten Biegebalkens;
- **Fig. 5A**  zeigt den in Fig. 5 dargestellten Biegebalken samt einem darauf aufgetragenen Wand-bzw. Deckenanstrich und applizierten Messpunkten;
- **Fig. 6**  in einer Draufsicht die gekerbte Breitseite eines für die zweite Prüfanordnung des erfindungsgemäßen Dehnmessverfahrens vorbereiteten Biegebalkens;
- **Fig. 6A**  zeigt den in Fig. 6 dargestellten Biegebalken samt einem darauf aufgetragenen Wand- bzw. Deckenbeschichtungssystem und applizierten Messpunkten.

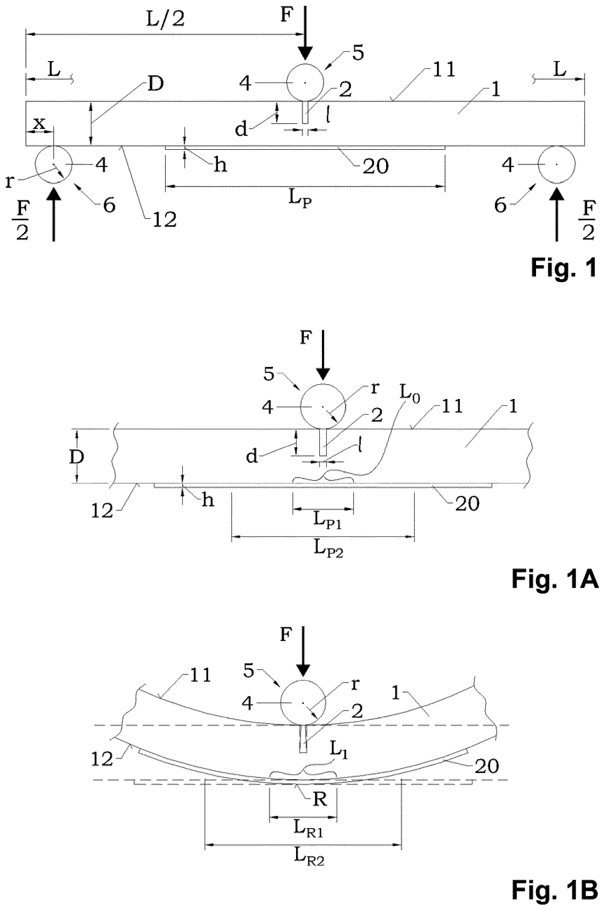

**Fig. 1** zeigt eine erste Prüfanordnung des Dehnmessverfahrens gemäß der Erfindung mit einem Biegebalken 1 zur Prüfung der Dehnfähigkeit eines Wand- bzw. Deckenanstrichs 20, der der Einfachheit halber im Folgenden kurz als Wandanstrich 20 bezeichnet wird, wie wohl dieser selbstredend auch zum Anstrich von anderen Gebäudeteilen wie beispielsweise zum Anstrich von Gebäudedecken eingesetzt werden kann.

Der Biegebalken 1 weist eine Balkenlänge L, eine Balkenbreite B sowie eine Balkendicke D bzw. Materialstärke auf. Der Biegebalken 1 ist hier beispielsweise aus einer zementgebundenen Spanplatte hergestellt, welche besonders vorteilhaft als saugfähiges Trägermaterial für einen Wandanstrich 20 geeignet ist. Beispielsweise hat der hier dargestellte Biegebalken 1 eine Balkenlänge L von rund 300 mm, eine Balkenbreite B von rund 75 mm sowie eine Balkendicke D bzw. Materialstärke von 24 mm. Gemäß der in Fig. 1 veranschaulichten ersten Prüfanordnung weist hier eine Breitseite 11 des Biegebalkens 1 in der Mitte ihrer Längsrichtung L/2 eine Kerbe 2 mit einer Kerbentiefe d sowie mit einer Kerbenbreite 1 auf. Die gekerbte Breitseite 11 bildet hier in Fig. 1 die Oberseite des Biegebalkens 1. Die Kerbentiefe d beträgt hier beispielsweise 50 % der Balkendicke D des Biegebalkens 1, somit also eine Kerbentiefe d von 12 mm. Die Kerbenbreite 1 wird in Längsrichtung L des Biegebalkens 1 gemessen und beträgt hier beispielsweise 3 mm. Die Kerbe 2 verläuft an der gekerbten Breitseite 11 des Biegebalkens 1 quer zu dessen Längsrichtung.

Der Biegebalken 1 wird beim erfindungsgemäßen Dehnmessverfahren in einem Drei-Punkt-Biegeversuch geprüft, wobei die drei Druckpunkte hier jeweils als Prüfstandsrollen 4 ausgeführt sind. Der Biegebalken 1 wird dazu auf zwei voneinander beabstandete Auflager 6, die hier als Prüfstandsrollen 4 ausgebildet sind, positioniert und während des Biegeversuchs in der Mitte zwischen den beiden Auflagern 6 mit einem Prüfstempel 5 mit einer Biegekraft F belastet und dabei während des Biegeversuchs deformiert. Die Biegekraft F wirkt entsprechend der Richtung des Pfeiles F lotrecht nach unten direkt im Bereich der Kerbe 2. Die Kerbe 2 dient zur Materialschwächung des Biegebalkens 1 und bewirkt, dass die Biegebeanspruchung des Biegebalkens 1 während des Biegeversuchs im Bereich der Kerbe 2 präzisiert wird und hier die Biegedeformation durch Stauchung der gekerbten Breitseite 11 besonders hoch ist. Somit wird erreicht, dass an der glatten Breitseite 12 des Biegebalkens 1, welche hier in Fig. 1 als Unterseite des Biegebalkens 1 gezeigt ist, in jenem Bereich, welcher der oberseitigen Kerbe 2 gegenüberliegt, die Dehnung an der glatten Breitseite 12 besonders hoch ist.

Gemäß der ersten Prüfanordnung wird die gekerbte Breitseite 11 während des Biegeversuchs mit dem Prüfstempel 5 belastet. Die der gekerbten Breitseite 11 gegenüberliegende Seite wird im Weiteren jeweils als glatte Breitseite 12 des Biegebalkens 1 bezeichnet. Die glatte Breitseite 12, welche in Fig. 1 als Unterseite des Biegebalkens 1 positioniert ist, ist während des Drei-Punkt-Biegeversuchs vom Prüfstempel 5 abgewandt und dient als Probeträgerseite für die zu prüfenden Wandanstriche 20 bzw. Wandbeschichtungen 25.

Im Sinne eines Kräftegleichgewichts während des Biegeversuchs wirkt jeweils die Hälfte der Biegekraft F/2 auf die beiden Auflager 6 ein. Die Prüfstandsrollen 4 sind hier jeweils als Metallwalzen ausgeführt und weisen jeweils einen Radius r auf. Die Auflager 6 werden dabei so eingestellt, dass zwischen der Rollenachse der die Auflager 6 bildenden Prüfstandsrollen 4 und dem seitlichen Balkenrand des Biegebalkens 1 ein Randabstand x an der glatten Breitseite 12 verbleibt, der hier dem Eineinhalbfachen des Radius r der Prüfstandsrollen 4 entspricht. Der Durchmesser der Prüfstandsrollen 4 ist hier so gewählt, dass dieser etwa der Balkendicke D bzw. Materialstärke des Biegebalkens 1 entspricht. Die Länge der Prüfstandsrollen 4 entspricht mindestens der Balkenbreite B des Biegebalkens 1.

Bei der in Fig. 1 skizzierten ersten Prüfanordnung des Dehnmessverfahrens wird an der glatten Breitseite 12 des Biegebalkens 1, welche gemäß Fig. 1 als Unterseite des Biegebalkens 1 der gekerbten Breitseite 11 an der Oberseite des Biegebalkens 1 gegenüber liegt, ein Wandanstrich 20 mit einer Schichthöhe h sowie mit einer Probenschichtlänge Lp aufgetragen. Der Bereich des Wandanstrichs 20 wird dabei so gewählt, dass die Probenschichtlänge Lp symmetrisch beiderseits der Längenmitte L/2 des Biegebalkens 1 aufgetragen wird und sich in Längsrichtung etwa über die Hälfte der Balkenlänge L des Biegebalkens 1 erstreckt. Oder in anderen Worten: Vom Balkenrand an den Schmalseiten des Biegebalkens 1 ausgehend verbleibt hier jeweils etwa ein Viertel der gesamten Balkenlänge L der glatten Breitseite 12 frei vom zu testenden Wandanstrich 20. Wesentlich ist jedoch, dass der Wandanstrich 20 im Bereich der Längenmitte L/2 des Biegebalkens 1 aufgetragen ist und damit dem Bereich der mit der Kerbe 2 versehenen gekerbten Breitseite 11 des Biegebalkens 1 gegenüberliegt.

**Fig. 1A** zeigt eine vergrößerte Detailansicht der in Fig. 1 gezeigten ersten Prüfanordnung in Ausgangslage mit dem noch unverformten Biegebalken 1. Vor Beginn des Dehnmessverfahrens werden auf dem Wandanstrich 20 Markierungen angeordnet, welche in Längsrichtung symmetrisch beiderseits der Längenmitte L/2 des Biegebalkens 1 aufgetragen werden. Die Längenmitte L/2 des Biegebalkens 1 entspricht der Schwächungszone der Kerbe 2 an der gegenüberliegenden gekerbten Breitseite 11 des Biegebalkens. Dazu werden auf dem Wandanstrich 20 zweckmäßig ein erstes Probenlängenintervall $L_{P1}$ sowie ein zweites Probenlängenintervall $L_{P2}$ aufgetragen. Das erste Probenlängenintervall $L_{P1}$ entspricht einem Zehntel der gesamten Biegebalkenlänge L. Das zweite Probenlängenintervall $L_{P2}$ entspricht einem Drittel der gesamten Biegebalkenlänge L. Anhand von Markierungen können Ausgangslängen $L_0$ vor Beginn der Einleitung einer Biegedeformation am noch unverformten Biegebalken 1 gemessen werden.

**Fig. 1B** zeigt die in Fig. 1A gezeigte erste Prüfanordnung nach einer Biegedeformation des Biegebalkens 1. Der Biegebalken 1 wird dazu in der Mitte zwischen den beiden Auflagern 6 mit dem Prüfstempel 5 mit einer Biegekraft F belastet und dabei während des Biegeversuchs deformiert. Aufgrund der Kerbe 2 an der gekerbten Breitseite 11 des Biegebalkens 1 wird die Deformation durch die Biegekraft F gezielt im Bereich der Kerbe 2 konzentriert. Die gekerbte Breitseite 11 des Biegebalkens 1 wird also im Bereich der Kerbe 2 besonders stark gestaucht. Wie vorhin bereits erwähnt, wird umgekehrt jener Bereich des Wandanstrichs 20 an der glatten Breitseite 12, der der Kerbe 2 gegenüberliegt, während der Verformung besonders stark gedehnt. Während der Deformation bildet sich beginnend in diesem besonders durch Dehnung belasteten Bereich an der glatten Breitseite 12 des Biegebalkens 1 eine Rissbildungszone R im Wandanstrich 20 aus. Besonders vorteilhaft treten somit während des Biegeversuchs erste Risse im Wandanstrich zuerst in diesem Abschnitt an der glatten Breitseite 12 des Biegebalkens 1 auf, welcher der Kerbe 2 gegenüberliegt. Diese Rissbildungszone R ist definiert als Zone mit ersten Haarrissen im Wandanstrich 20, welche eine Rissbreite von größer oder gleich 0,2 mm aufweisen und somit bereits mit freiem Auge als Risse im Wandanstrich 20 erkennbar sind.

Um die Rissbildung möglichst in Echtzeit beobachten zu können, wird zweckmäßig eine hochauflösende Industriekamera zur Dokumentation des Biegeversuchs eingesetzt. Dabei werden auch die Längenänderungen der zuvor erfassten Ausgangslängen $L_0$ bzw. der vorerfassten Längen der Probenlängenintervalle $L_{P1}$, $L_{P2}$ von der Industriekamera aufgezeichnet. Vorteilhaft können die ursprünglich erfassten Längenmaße und Ausgangslängen im unverformten Zustand vor der Biegedeformation des Biegebalkens 1 mit jenen vergrößerten Probenlängenintervallen $L_{R1}$, $L_{R2}$ zum Zeitpunkt bei bereits erkennbarer, beginnender Rissbildung in der Rissbildungszone R nach erfolgter Biegedeformation des Biegebalkens 1 verglichen werden. Das erste Probenlängenintervall $L_{R1}$ bei bereits aufgetretener Rissbildung nach der Biegedeformation entspricht einem durch Biegedeformation vergrößerten ersten Probenlängenintervall $L_{P1}$. Analog dazu entspricht das zweite Probenlängenintervall $L_{R2}$ bei bereits aufgetretener Rissbildung nach der Biegedeformation des Biegebalkens 1 einem aufgrund der Biegedeformation vergrößerten zweiten Probenlängenintervall $L_{P2}$. Mit diesen in der ersten Messanordnung erhaltenen Messdaten kann die Dehnfähigkeit des geprüften Wandanstrichs 20 bestimmt werden.

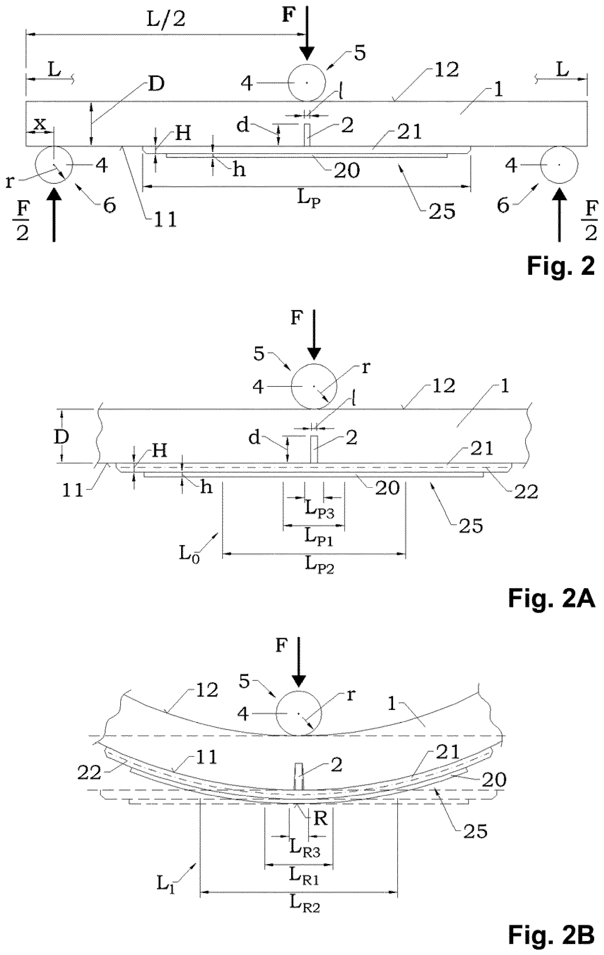

**Fig. 2** zeigt eine zweite Prüfanordnung des Dehnmessverfahrens gemäß der Erfindung mit einem Biegebalken 1 zur Prüfung der Dehnfähigkeit sowie des Rissüberbrückungsvermögens eines mehrlagigen Wand- bzw. Deckenbeschichtungssystems 25, welches hier eine Spachtelmasse 21 sowie einen auf der Spachtelmasse 21 angebrachten Wandanstrich 20 umfasst. Das Wand- bzw. Deckenbeschichtungssystem 25 wird im Folgenden kurz als Wandbeschichtungssystem 25 bezeichnet, wie wohl dieses auch zum Beschichten anderer Gebäudeteile wie beispielsweise Gebäudedecken geeignet ist. Gemäß der in Fig. 2 veranschaulichten zweiten Prüfanordnung weist hier die gekerbte Breitseite 11 des Biegebalkens 1 in der Längenmitte L/2 quer zu ihrer Längsrichtung eine Kerbe 2 mit einer Kerbentiefe d sowie mit einer Kerbenbreite 1 auf. Die Kerbentiefe d beträgt hier beispielsweise 50 % der Balkendicke D des Biegebalkens 1, somit also eine Kerbentiefe d von 12 mm. Die Kerbenbreite 1 wird in Längsrichtung L des Biegebalkens 1 gemessen und beträgt hier beispielsweise 3 mm. Die Kerbe 2 verläuft an der gekerbten Breitseite 11 des Biegebalkens 1 quer zu dessen Längsrichtung. Die gekerbte Breitseite 11 befindet in Fig. 2 an der Unterseite des Biegebalkens 1. Die Kerbe 2 dient auch hier zur gezielten Materialschwächung des Biegebalkens 1, um während des Biegeversuchs im Bereich der Kerbe 2 eine besonders starke Dehnung der gekerbten Breitseite 11 zu erzielen. Weiters dient die Kerbe 2 in der zweiten Prüfanordnung als Modell eines künstlich erzeugten Risses einer Gebäudewand oder Gebäudedecke, welcher vor dem Auftrag eines Wandanstrichs 20 mit einer Spachtelmasse 21 überdeckt wird.

Dazu wird in Vorbereitung des Biegeversuchs auf der gekerbten Breitseite 11 des unverformten Biegebalkens 1, also auf jener Seite, welche die Kerbe 2 trägt und die im nachfolgenden Biegeversuch dem Prüfstempel 5 gegenüberliegt, zuerst eine Spachtelmasse 21 mit einer Schichthöhe H bzw. Schichtdicke satt aufgetragen. Mit der Spachtelmasse 21 wird dabei auch die Kerbe 2 aufgefüllt. Dazu wird zuerst die Kerbe 2 plan ausgefüllt und anschließend nach Aushärtung der Spachtelmasse 21 in der Kerbe 2 die weitere Spachtelschicht 21 aufgetragen. Die Spachtelmasse 21 wird dabei flächig in Längsrichtung L des Biegebalkens 1 zumindest entlang eines Abschnittes mit einer Probenschichtlänge $L_P$ aufgetragen. Nach dem Aushärten der Spachtelmasse 21 erfolgt anschließend der Auftrag eines Wandanstrichs 20 mit einer Schichthöhe h bzw. Schichtdicke auf der bereits ausgehärteten Spachtelmasse 21.

Die Kombination aus einer Schicht einer Spachtelmasse 21 und einer oder auch mehreren Lagen eines Wandanstrichs 20 wird als Wandbeschichtungssystem 25 bezeichnet.

**Fig. 2A** stellt die in Fig. 2 gezeigte zweite Prüfanordnung in Ausgangslage mit einem unverformten Biegebalken 1 dar. In Fig. 2A ist eine Armierungslage 22 aus einem Glasfaservlies innerhalb der Schichthöhe H der Spachtelmasse 21 angedeutet. Ein Wandbeschichtungssystem 25 kann beispielsweise auch eine solche Armierungslage 22 umfassen, die zweckmäßig als Glasfaservlies ausgeführt ist und üblicherweise etwa mittig der Schichthöhe H bzw. Schichtdicke in die Spachtelmasse 21 eingelegt wird. Dazu wird zuerst eine dünne Schicht der Spachtelmasse 21 aufgetragen, welche die Kerbe 2 überdeckt. Anschließend kann die Armierungslage 22 aufgelegt und diese mit einer weiteren dünnen Schicht der Spachtelmasse 21 überdeckt werden, um somit eine armierte Spachtelmasse 21 mit einer Schichthöhe H zu erhalten.

**Fig. 2B** stellt die in Fig. 2A gezeigte zweite Prüfanordnung nach einer Biegedeformation des Biegebalkens 1 dar. Wie bereits zuvor anhand der Fig. 1B beschrieben, wird auch gemäß Fig. 2B der Biegebalken 1 in der Mitte zwischen den beiden Auflagern 6 mit dem Prüfstempel 5 mit einer Biegekraft F belastet und dabei während des Biegeversuchs deformiert. Aufgrund der Kerbe 2 an der gekerbten Breitseite 11 des Biegebalkens 1 wird die Deformation durch die Biegekraft F gezielt im Bereich der Kerbe 2 konzentriert. Die glatte Breitseite 12 des Biegebalkens 1, die in Fig. 2B die Oberseite des Biegebalkens bildet, wird also im Bereich der Kerbe 2 besonders stark gestaucht. Jener Bereich des Wandbeschichtungssystems 25 an der gekerbten Breitseite 11 im Bereich der Kerbe 2 wird während der Verformung besonders stark gedehnt. Während der Deformation bildet sich in diesem besonders durch Dehnung belasteten Bereich an der gekerbten Breitseite 11 des Biegebalkens 1 eine Rissbildungszone R im Wandbeschichtungssystem 25 aus. Besonders vorteilhaft treten somit während des Biegeversuchs erste Risse im Wandanstrich 20 bzw. in der darunter aufgetragenen Spachtelmasse 21 zuerst in diesem Abschnitt im Bereich der Kerbe 2 an der Unterseite des Biegebalkens 1 auf. Diese Rissbildungszone R ist abermals definiert als Zone mit ersten Haarrissen in einer oder mehrerer Lagen des Wandbeschichtungssystems 25. Haarrisse weisen hier definitionsgemäß eine Rissbreite von größer oder gleich 0,2 mm auf und sind somit bereits mit freiem Auge als Risse erkennbar.

Um die Rissbildung möglichst in Echtzeit beobachten zu können, wird zweckmäßig auch hier bei der zweiten Prüfanordnung eine Industriekamera zur Dokumentation des Biegeversuchs eingesetzt. Analog zur ersten Prüfanordnung werden auch hier die Längenänderungen der zuvor erfassten Ausgangslängen $L_0$ bzw. Probenlängenintervalle $L_{P1}$, $L_{P2}$ $L_{P3}$ von der Industriekamera aufgezeichnet. Dazu werden auf dem zu prüfenden Wandbeschichtungssystem 25 - analog zur ersten Prüfanordnung - zweckmäßig ein erstes Probenlängenintervall $L_{P1}$ sowie ein zweites Probenlängenintervall $L_{P2}$ aufgetragen. Das erste Probenlängenintervall $L_{P1}$ entspricht - analog zur ersten Prüfanordnung - wiederum einem Zehntel der gesamten Biegebalkenlänge L. Das zweite Probenlängenintervall $L_{P2}$ entspricht wiederum einem Drittel der gesamten Biegebalkenlänge L. Überdies wird im Rahmen der Prüfung des Wandbeschichtungssystems 25 noch ein weiteres, drittes Probenlängenintervall $L_{P3}$ aufgetragen, welches den Probenlängenabschnitt der Messpunkte, die direkt an die Kerbe 2 angrenzen, angibt. Das dritte Probenlängenintervall $L_{P3}$ entspricht somit der Kerbenbreite 1 sowie dem zweifachen Wert der halben Abstandbreite des Abstands eines Messpunkts von der Kerbe 2. Anhand von entsprechenden Messpunkt-Markierungen können die Ausgangslängen $L_0$ vor Beginn der Einleitung einer Biegedeformation am noch unverformten Biegebalken 1 gemessen werden.

Vorteilhaft können die ursprünglich erfassten Längenmaße und Ausgangslängen im unverformten Zustand vor der Biegedeformation des Biegebalkens 1 mit jenen vergrößerten Probenlängenintervallen $L_{R1}$, $L_{R2}$, $L_{R3}$ zum Zeitpunkt bei beginnender Rissbildung in der Rissbildungszone R nach erfolgter Biegedeformation des Biegebalkens 1 verglichen werden. Das erste Probenlängenintervall $L_{R1}$ entspricht wiederum dem Probenlängenintervall $L_{P1}$ bei bereits aufgetretener Rissbildung nach der Biegedeformation. Das zweite Probenlängenintervall $L_{R2}$ entspricht dem Probenlängenintervall $L_{P2}$ bei bereits aufgetretener Rissbildung nach der Biegedeformation des Biegebalkens 1. Ebenso zeigt das dritte Probenlängenintervall $L_{R3}$ bei bereits aufgetretener Rissbildung nach der Biegedeformation des Biegebalkens 1 die Änderung der Kerbenbreite 1 gegenüber dem ursprünglichen Probenlängenintervall $L_{P3}$ am unverformten Biegebalken 1. Mit diesen gemäß der zweiten Prüfanordnung erhaltenen Messdaten lassen sich die Dehnfähigkeit sowie das Rissüberbrückungsvermögen des geprüften Wandbeschichtungssystems 25 bestimmen.

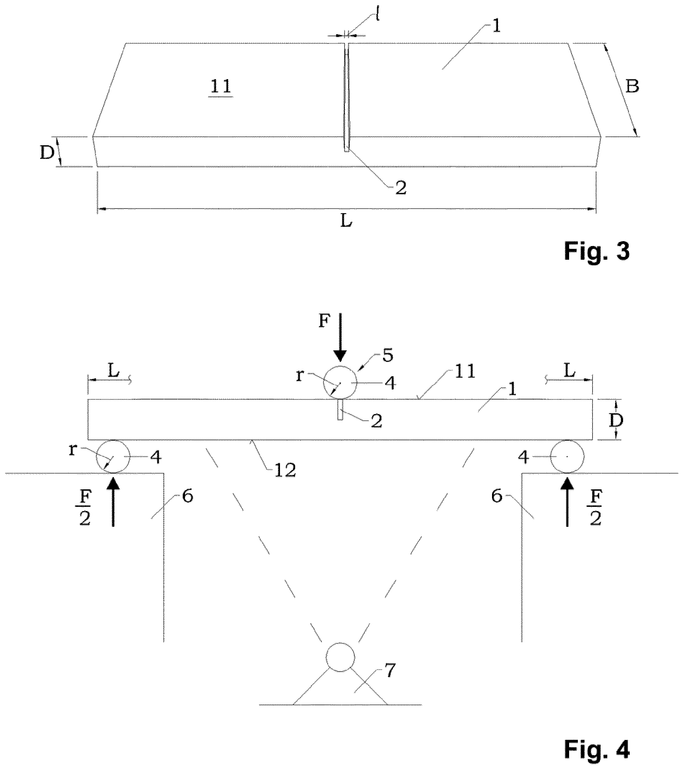

**Fig. 3** veranschaulicht einen Biegebalken 1 mit einer Kerbe 2 quer zur Längsrichtung L des Biegebalkens 1. Der hier gezeigte Biegebalken 1 ist aus einer zementgebundenen Spanplatte hergestellt, welche besonders vorteilhaft als saugfähiges Trägermaterial für den Auftrag eines zu prüfenden Wandanstrichs 20 und/oder eines Wandbeschichtungssystems 25 geeignet ist.

**Fig. 4** zeigt in einer schematischen Seitenansicht den Prüfaufbau eines Biegebalken-Dehnmessverfahrens gemäß der ersten Prüfanordnung unter Einsatz einer hochauflösenden Industriekamera 7. Die Industriekamera 7 ist dazu beispielsweise unterhalb des Biegebalkens 1 sowie von diesem beabstandet im Bereich zwischen den beiden Auflager 6 montiert. In dieser Lage gewährleistet die Industriekamera 7 einen uneingeschränkten Blick auf die glatte Breitseite 12 des Biegebalkens 1 samt dem zu prüfenden Wandanstrich 20. Wie vorhin bereits dargelegt, wird gemäß der ersten Prüfanordnung der Biegebalken 1 mit dem zu prüfenden Wandanstrich 20 so vorbereitet, dass der Wandanstrich 20 an der glatten Breitseite 12 des Biegebalkens 1 aufgetragen wird. Der Biegebalken 1 wird nach dem Aushärten des Wandanstrichs 20 sowie nachdem entsprechende Probenlängenintervalle $L_{P1}$, $L_{P2}$, $L_{P3}$ mittels Messpunkten M am Wandanstrich 20 angeordnet wurden, so in der Prüfvorrichtung positioniert, dass der Wandanstrich 20 zur Industriekamera 7 hin orientiert ist. Die neben der Industriekamera 7 zur Messdatenerfassung und -auswertung weiteren Komponenten - also ein entsprechender Messdaten-Computer zur Erfassung, Speicherung und Auswertung der Messdaten sowie der gegebenenfalls benötigten Verkabelung zur Signalverbindung der einzelnen Komponenten - die gemeinsam einen sogenannten Videoextensometer bilden, sind der Einfachheit halber in Fig. 4 nicht explizit dargestellt. Die Industriekamera 7 symbolisiert daher hier auch weitere erforderliche Komponenten und Geräte, die dem Fachmann bekannt sind und die gemeinsam als Videoextensometer zur Erfassung von Dehnungsmessungen eingesetzt werden.

Mit diesem Prüfaufbau, wie dieser in Fig. 4 dargestellt ist, kann selbstredend auch ein gemäß der zweiten Prüfanordnung zu prüfendes Wandbeschichtungssystem 25 vermessen werden. Die dazu benötigte Anordnung des Biegebalkens 1 entspricht der Ansicht von Fig. 2. Wie vorhin bereits dargelegt, wird gemäß der zweiten Prüfanordnung der Biegebalken 1 mit dem zu prüfenden Wandbeschichtungssystem 25 so vorbereitet, dass das Wandbeschichtungssystem 25 an der gekerbten Breitseite 11 des Biegebalkens 1 aufgetragen wird. Der Biegebalken 1 wird nach dem Aushärten des Wandbeschichtungssystems 25 sowie nachdem entsprechende Probenlängenintervalle $L_{P1}$, $L_{P2}$, $L_{P3}$ mittels Messpunkten M am Wandbeschichtungssystem 25 angeordnet wurden, so in der Prüfvorrichtung positioniert, dass das Wandbeschichtungssystem 25 zur Industriekamera 7 hin orientiert ist.

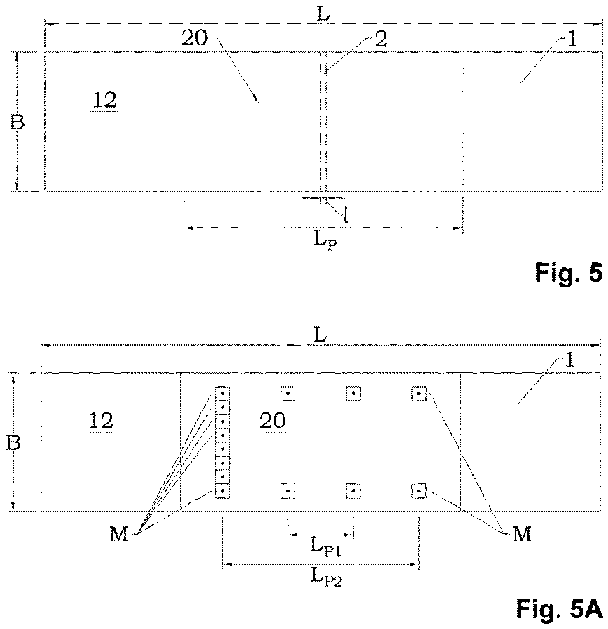

**Fig. 5** zeigt die glatte Breitseite 12 eines für die erste Prüfanordnung des erfindungsgemäßen Dehnmessverfahrens vorbereiteten Biegebalkens 1. Die Ansicht von Fig. 5 entspricht daher der Untersicht des in Fig. 1 veranschaulichten Biegebalkens 1. Die Kerbe 2 ist hier in Fig. 5 an der Rückseite, also an der der glatten Breitseite 12 gegenüberliegenden gekerbten Breitseite 11 des Biegebalkens 1 angeordnet.

**Fig. 5A** zeigt den in Fig. 5 dargestellten Biegebalken 1 samt dem auf der glatten Breitseite 12 entlang einer Probenschichtlänge $L_P$ darauf aufgetragenen Wandanstrich 20. Es sind auf dem Wandanstrich mehrere Messpunkte M zum Detektieren der Längenänderungen der damit erfassten Ausgangslängen $L_0$, entsprechend den Probenlängenintervallen $L_{P1}$ bzw. $L_{P2}$, mit einer Industriekamera angebracht. Vorteilhaft können die ursprünglich erfassten Ausgangslängen $L_0$ im unverformten Zustand vor der Biegedeformation des Biegebalkens 1 mit jenen Zustandslängen $L_1$ der durch Biegedeformation vergrößerten Probenlängenintervalle $L_{R1}$, $L_{R2}$ zum Zeitpunkt bei erfolgter Rissbildung in der Rissbildungszone R des Biegebalkens 1 verglichen werden.

Es wird solcherart die durch die Biegedeformation verursachte Längenänderung $\Delta_L$ des am Biegebalkens 1 aufgetragenen Wandanstrichs 20 bestimmt. Anschließend kann die Dehnfähigkeit $\varepsilon$ des getesteten Wandanstrichs 20 anhand des vorhin dargelegten Berechnungsschemas ermittelt werden.

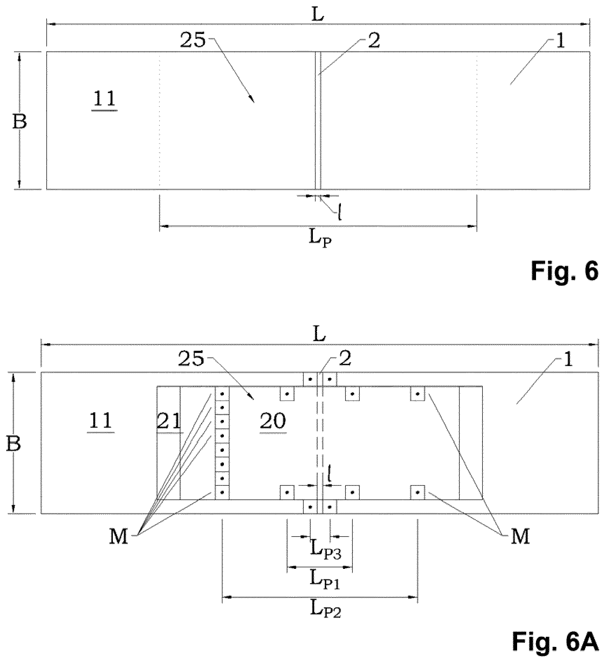

**Fig. 6** zeigt die gekerbte Breitseite 11 eines für die zweite Prüfanordnung des erfindungsgemäßen Dehnmessverfahrens vorbereiteten Biegebalkens 1. Die Ansicht von Fig. 6 entspricht daher der Untersicht des in Fig. 2 veranschaulichten Biegebalkens 1. Die Kerbe 2 ist hier in Fig. 6 an der sichtbaren Breitseite 11 des Biegebalkens 1 angeordnet.

**Fig. 6A** zeigt den in Fig. 6 dargestellten Biegebalken 1 samt dem auf der gekerbten Breitseite 11 entlang einer Probenschichtlänge $L_P$ darauf aufgetragenen Wandbeschichtungssystem 25, welches hier beispielsweise eine Schicht einer Spachtelmasse 21, welche von einem Wandanstrich 20 bedeckt ist, umfasst. Es sind auf dem Wandanstrich 20 abermals mehrere Messpunkte M zum Detektieren der Längenänderungen der zuvor erfassten Ausgangslängen $L_0$ bzw. Probenlängenintervalle $L_{P1}$, $L_{P2}$, $L_{P3}$ mit einer Industriekamera angebracht. Vorteilhaft können die ursprünglich erfassten Längenmaße und Ausgangslängen im unverformten Zustand vor der Biegedeformation des Biegebalkens 1 mit jenen vergrößerten Zustandslängen $L_1$ der Probenlängenintervalle $L_{R1}$, $L_{R2}$, $L_{R3}$ nach erfolgter Biegedeformation des Biegebalkens 1, also bei bereits aufgetretener Rissbildung in der Rissbildungszone R, verglichen werden.

Es wird solcherart die durch die Biegedeformation verursachte Längenänderung $\Delta_L$ des am Biegebalkens 1 aufgetragenen Wandbeschichtungssystems 25 bestimmt. Anschließend kann die Dehnfähigkeit $\varepsilon$ des getesteten mehrlagigen Wandbeschichtungssystems 25 durch Berechnung ermittelt werden. Ebenso kann das Rissüberbrückungsvermögen des getesteten mehrlagigen Wandbeschichtungssystems 25 anhand des vorhin dargelegten Berechnungsschemas ermittelt werden.

LISTE DER BEZUGSZEICHEN

| 1 | Biegebalken |
|---|---|
| 2 | Kerbe |
| 4 | Prüfstandsrolle |
| 5 | Prüfstempel |
| 6 | Auflager |
| 7 | Industriekamera |
| 11 | gekerbte Breitseite des Biegebalkens |
| 12 | glatte Breitseite des Biegebalkens |
| 20 | Wand- bzw. Deckenanstrich |
| 21 | Spachtelmasse |
| 22 | Armierungslage |
| 25 | Wandbeschichtungssystem |
| B | Balkenbreite des Biegebalkens |
| D | Balkendicke bzw. Materialstärke des Biegebalkens |
| d | Kerbentiefe |
| F | Biegekraft |
| F/2 | Hälfte der Biegekraft |
| H | Schichthöhe bzw. Schichtdicke der Spachtelmasse |
| h | Schichthöhe bzw. Schichtdicke des Wandanstrichs |
| L | Balkenlänge des Biegebalkens |
| L/2 | Längenmitte des Biegebalkens |
| l | Kerbenbreite in Längsrichtung des Biegebalkens |
| $L_P$ | Probenschichtlänge |
| $L_0$ | Ausgangslänge vor der Biegeformation, entspricht $L_{P1}$, $L_{P2}$ bzw. $L_{P3}$ |
| $L_1$ | Zustandslänge bei Rissbildung, entspricht $L_{R1}$, $L_{R2}$ bzw. $L_{R3}$ |
| $L_{P1}$ | Probenlängenintervall 1 vor der Biegedeformation; 1/10 der Biegebalkenlänge L |
| $L_{P2}$ | Probenlängenintervall 2 vor der Biegedeformation; 1/3 der Biegebalkenlänge L |
| $L_{P3}$ | Probenlängenintervall 3 vor der Biegedeformation |
| $L_{R1}$ | Probenlängenintervall 1 bei Rissbildung nach Biegedeformation |
| $L_{R2}$ | Probenlängenintervall 2 bei Rissbildung nach Biegedeformation |
| $L_{R3}$ | Probenlängenintervall 3 bei Rissbildung nach Biegedeformation |
| M | Messpunkt |
| R | Rissbildungszone; Zone mit Haarrissen |
| r | Radius der Prüfstandsrolle |
| x | Randabstand |

**Patentansprüche**

1. Dehnmessverfahren zur Bestimmung der Dehnfähigkeit dünner Schichten eines Wandanstrichs (20) oder eines mehrlagigen Wandbeschichtungssystems (25), **gekennzeichnet durch** die folgenden Schritte:

    -a- Bereitstellen eines Biegebalkens (1) aus einem saugfähigen Material, wobei der Biegebalken (1) eine Balkenlänge (L) sowie einen rechteckigen Querschnitt mit einer Balkenbreite (B) sowie mit einer Balkendicke (D) aufweist;

    -b- Anordnen einer Kerbe (2) auf einer Breitseite (11) des Biegebalkens (1) in der Längenmitte (L/2) seiner Balkenlänge (L) sowie quer zur Längsrichtung des Biegebalkens (1), wobei die Kerbe (2) eine Kerbentiefe (d) sowie eine Kerbenbreite (1) aufweist und über die gesamte Balkenbreite (B) verläuft;

    wahlweise - nach einer ersten Prüfanordnung -

    -c1- Auftragen eines Wandanstrichs (20) entlang eines Abschnitts mit einer Probenschichtlänge ($L_P$) an der der gekerbten Breitseite (11) gegenüberliegenden glatten Breitseite (12) des Biegebalkens (1) beiderseits der Längenmitte (L/2) seiner Balkenlänge (L);

    oder - alternativ nach einer zweiten Prüfanordnung -

    -c2- Auftragen eines mehrlagigen Wandbeschichtungssystems (25) entlang eines Abschnitts mit einer Probenschichtlänge ($L_P$) an der gekerbten Breitseite (11) des Biegebalkens (1) beiderseits der Längenmitte (L/2) seiner Balkenlänge (L);

    sowie weiters

    -d- Anordnen von Messpunkten (M) in zumindest einem Probenlängenintervall ($L_{P1}$, $L_{P2}$, $L_{P3}$) der Probenschichtlänge ($L_P$) des aufgetragenen Wandanstrichs (20) oder des mehrlagigen Wandbeschichtungssystems (25);

    -e- Aufzeichnen zumindest einer Ausgangslänge ($L_0$) als Abstandsmaß zwischen Messpunkten (M) des zumindest einen Probenlängenintervalls ($L_{P1}$, $L_{P2}$, $L_{P3}$) am unverformten Biegebalken (1);

    -f- Durchführen eines Drei-Punkt-Biegeversuchs mit einer Prüfvorrichtung, wobei der Biegebalken (1) auf zwei seitlichen Auflagern (6) derart positioniert wird, dass die mit dem Wandanstrich (20) oder dem mehrlagigen Wandbeschichtungssystem (25) beschichtete Seite zu den seitlichen Auflagern (6) hin orientiert ist sowie einem in Längenmitte (L/2) des Biegebalkens (1) mittigen Prüfstempel (5) gegenüberliegt;

    -g- Aufzeichnen der Biegedeformation des Biegebalkens (1) während des Drei-Punkt-Biegeversuchs mit einer Industriekamera (7), die die dem mittigen Prüfstempel (5) gegenüberliegende beschichtete Seite des Biegebalkens (1) erfasst;

    -h- Beendigen des Drei-Punkt-Biegeversuchs, sobald in einer Rissbildungszone (R) der beschichteten Seite des Biegebalkens (1) eine Rissbildung mit Haarrissen im Wandanstrich (20) oder im mehrlagigen Wandbeschichtungssystem (25) detektiert wird;

    -i- Ermitteln der Längenänderung $\Delta_L$ des am Biegebalken (1) aufgetragenen Wandanstrichs (20) oder Wandbeschichtungssystems (25) als Differenz zumindest einer Zustandslänge ($L_1$) bei Rissbildung, welche Zustandslänge ($L_1$) als Abstandsmaß zwischen Messpunkten (M) des zumindest einen Probenlängenintervalls ($L_{R1}$, $L_{R2}$, $L_{R3}$) bei Rissbildung am deformierten Biegebalken (1) erhalten wird, und der Ausgangslänge ($L_0$) des zumindest einen Probenlängenintervalls ($L_{P1}$, $L_{P2}$, $L_{P3}$) am unverformten Biegebalken (1).

2. Dehnmessverfahren nach Anspruch 1, **dadurch gekennzeichnet, dass** die Dehnfähigkeit $\varepsilon$ des getesteten Wandanstrichs (20) oder des mehrlagigen Wandbeschichtungssystems (25) als Verhältnis der Längenänderung $\Delta_L$ des Biegebalkens (1) zur ursprünglichen Ausgangslänge ($L_0$) berechnet wird, wobei die Längenänderung $\Delta_L$ als Differenz von deformierter Zustandslänge ($L_1$) bei Rissbildung zur ursprünglichen Ausgangslänge ($L_0$) ausgedrückt wird.

3. Dehnmessverfahren nach Anspruch 1 oder 2, **dadurch gekennzeichnet, dass** das Rissüberbrückungsvermögen des getesteten mehrlagigen Wandbeschichtungssystems (25) als Differenz von deformierter Zustandslänge ($L_1$) des Probenlängenintervalls ($L_{R3}$) bei Rissbildung, welches Probenlängenintervall ($L_{R3}$) direkt an die Kerbe (2) angrenzende Messpunkte (M) aufweist, zur ursprünglichen Ausgangslänge ($L_0$) dieses Probenlängenintervalls ($L_{P3}$) mit direkt an die Kerbe (2) angrenzenden Messpunkten (M), berechnet wird.

4. Dehnmessverfahren nach einem der Ansprüche 1 bis 3, **dadurch gekennzeichnet, dass** die deformierte Zustandslänge ($L_1$) bei Rissbildung von Haarrissen im Wandanstrich (20) oder im mehrlagigen Wandbeschichtungssystem (25) mit einer Rissöffnung von zumindest 0,2 mm bestimmt wird.

5. Dehnmessverfahren nach einem der Ansprüche 1 bis 4, **dadurch gekennzeichnet, dass** ein Biegebalken (1) bereitgestellt wird, welcher eine Balkendicke (D) von 10 mm bis 40 mm, bevorzugt von 20 mm bis 28 mm, besonders bevorzugt von 24 mm, aufweist.

6. Dehnmessverfahren nach einem der Ansprüche 1 bis 5, **dadurch gekennzeichnet, dass** ein Biegebalken (1) bereitgestellt wird, welcher eine Balkenbreite (B) aufweist, die dem dreifachen Wert der Balkendicke (D) entspricht, sowie eine Balkenlänge (L) aufweist, die dem zwölffachen Wert der Balkendicke (D) entspricht.

7. Dehnmessverfahren nach einem der Ansprüche 1 bis 6, **dadurch gekennzeichnet, dass** ein Biegebalken (1) bereitgestellt wird, welcher eine Kerbe (2) mit einer Kerbentiefe (d) aufweist, die von 20 % bis 80 % der Balkendicke (D), bevorzugt von 40 % bis 60 % der Balkendicke (D), besonders bevorzugt 50 % der Balkendicke (D), beträgt.

8. Dehnmessverfahren nach einem der Ansprüche 1 bis 7, **dadurch gekennzeichnet, dass** ein Biegebalken (1) bereitgestellt wird, welcher eine Kerbe (2) mit einer Kerbenbreite (1) von 2 mm bis 4 mm aufweist.

9. Dehnmessverfahren nach einem der Ansprüche 1 bis 8, **dadurch gekennzeichnet, dass** ein Biegebalken (1) bereitgestellt wird, welcher aus einer Komposit-Platte enthaltend Holz und Zement hergestellt ist.

10. Dehnmessverfahren nach Anspruch 9, **dadurch gekennzeichnet, dass** der bereitgestellte Biegebalken (1)

   - von 50 Vol.-% bis 70 Vol.-% an Holz oder Holzspänen,
   - von 20 Vol.-% bis 40 Vol.-% an Zement,
   - von 7 Vol.-% bis 15 Vol.-% an gebundenem Wasser sowie
   - von 0 Vol.-% bis 3 Vol.-% an zusätzlichen Hydratationszusätzen enthält.

11. Prüfaufbau zur Durchführung eines Dehnmessverfahrens nach einem der Ansprüche 1 bis 10 zur Bestimmung der Dehnfähigkeit dünner Schichten eines Wandanstrichs (20) nach einer ersten Prüfanordnung, **dadurch gekennzeichnet, dass**

   - der Prüfaufbau einen Biegebalken (1) sowie eine mit einer Industriekamera (7) sowie mit einer Mess-Software ausgestattete Prüfvorrichtung zur Durchführung eines Drei-Punkt-Biegeversuchs umfasst, wobei die Industriekamera dazu ausgebildet ist, die Verformung am zu prüfenden Biegebalken (1) während des Biegevorgangs aufzuzeichnen, und die Mess-Software dazu ausgebildet ist, die aufgezeichneten Verformungen und Verschiebungen zu speichern und auszuwerten; wobei
   - der Biegebalken (1) aus einem saugfähigen Material bereitgestellt ist, eine Balkenlänge (L) sowie einen rechteckigen Querschnitt mit einer Balkenbreite (B) und mit einer Balkendicke (D) aufweist, wobei eine Kerbe (2) auf einer Breitseite (11) des Biegebalkens (1) in der Längenmitte (L/2) seiner Balkenlänge (L) sowie quer zur Längsrichtung des Biegebalkens (1) angeordnet ist, wobei die Kerbe (2) eine Kerbentiefe (d) sowie eine Kerbenbreite (1) aufweist und über die gesamte Balkenbreite (B) verläuft; sowie
   - entlang eines Abschnitts mit einer Probenschichtlänge ($L_P$) an der der gekerbten Breitseite (11) gegenüberliegenden glatten Breitseite (12) des Biegebalkens (1) beiderseits der Längenmitte (L/2) seiner Balkenlänge (L) ein Wandanstrich (20) aufgetragen ist; sowie weiters
   - Messpunkte (M) in zumindest einem Probenlängenintervall ($L_{P1}$, $L_{P2}$, $L_{P3}$) der Probenschichtlänge ($L_P$) des aufgetragenen Wandanstrichs (20) angeordnet sind,
   - wobei zumindest eine Ausgangslänge ($L_0$) als Abstandsmaß zwischen Messpunkten (M) des zumindest einen Probenlängenintervalls ($L_{P1}$, $L_{P2}$, $L_{P3}$) am unverformten Biegebalken (1) aufgezeichnet ist;
   - wobei der Biegebalken (1) zur Durchführung eines Drei-Punkt-Biegeversuchs auf zwei seitlichen Auflagern (6) der Prüfvorrichtung derart positioniert ist, dass die mit dem Wandanstrich (20) beschichtete Seite zu den seitlichen Auflagern (6) hin orientiert ist sowie einem in Längenmitte (L/2) des Biegebalkens (1) mittig angeordneten Prüfstempel (5) der Prüfvorrichtung gegenüberliegt; wobei
   - die Aufzeichnung der Biegedeformation des Biegebalkens (1) während des Drei-Punkt-Biegeversuchs mit einer Industriekamera (7) erfolgt, welche Industriekamera (7) die dem mittigen Prüfstempel (5) gegenüberliegende beschichtete Seite des Biegebalkens (1) erfasst; wobei
   - die Prüfvorrichtung derart konfiguriert ist, dass der Drei-Punkt-Biegeversuch endet, sobald die Industriekamera (7) in einer Rissbildungszone (R) der beschichteten Seite des Biegebalkens (1) eine Rissbildung mit Haarrissen im Wandanstrich (20) detektiert.

12. Prüfaufbau zur Durchführung eines Dehnmessverfahrens nach einem der Ansprüche 1 bis 10 zur Bestimmung der Dehnfähigkeit dünner Schichten eines mehrlagigen Wandbeschichtungssystems (25) nach einer zweiten Prüfanordnung,

**dadurch gekennzeichnet, dass**

- der Prüfaufbau einen Biegebalken (1) sowie eine mit einer Industriekamera (7) sowie mit einer Mess-Software ausgestattete Prüfvorrichtung zur Durchführung eines Drei-Punkt-Biegeversuchs umfasst, wobei die Industriekamera dazu ausgebildet ist, die Verformung am zu prüfenden Biegebalken (1) während des Biegevorgangs aufzuzeichnen, und die Mess-Software dazu ausgebildet ist, die aufgezeichneten Verformungen und Verschiebungen zu speichern und auszuwerten; wobei

- der Biegebalken (1) aus einem saugfähigen Material bereitgestellt ist, eine Balkenlänge (L) sowie einen rechteckigen Querschnitt mit einer Balkenbreite (B) und mit einer Balkendicke (D) aufweist, wobei eine Kerbe (2) auf einer Breitseite (11) des Biegebalkens (1) in der Längenmitte (L/2) seiner Balkenlänge (L) sowie quer zur Längsrichtung des Biegebalkens (1) angeordnet ist, wobei die Kerbe (2) eine Kerbentiefe (d) sowie eine Kerbenbreite (1) aufweist und über die gesamte Balkenbreite (B) verläuft; sowie

- entlang eines Abschnitts mit einer Probenschichtlänge ($L_P$) an der gekerbten Breitseite (11) des Biegebalkens (1) beiderseits der Längenmitte (L/2) seiner Balkenlänge (L) ein mehrlagiges Wandbeschichtungssystem (25) aufgetragen ist; sowie weiters

- Messpunkte (M) in zumindest einem Probenlängenintervall ($L_{P1}$, $L_{P2}$, $L_{P3}$) der Probenschichtlänge ($L_P$) des aufgetragenen Wandanstrichs (20) angeordnet sind,

- wobei zumindest eine Ausgangslänge ($L_0$) als Abstandsmaß zwischen Messpunkten (M) des zumindest einen Probenlängenintervalls ($L_{P1}$, $L_{P2}$, $L_{P3}$) am unverformten Biegebalken (1) aufgezeichnet ist;

- wobei der Biegebalken (1) zur Durchführung eines Drei-Punkt-Biegeversuchs auf zwei seitlichen Auflagern (6) der Prüfvorrichtung derart positioniert ist, dass die mit dem Wandanstrich (20) beschichtete Seite zu den seitlichen Auflagern (6) hin orientiert ist sowie einem in Längenmitte (L/2) des Biegebalkens (1) mittig angeordneten Prüfstempel (5) der Prüfvorrichtung gegenüberliegt; wobei

- die Aufzeichnung der Biegedeformation des Biegebalkens (1) während des Drei-Punkt-Biegeversuchs mit einer Industriekamera (7) erfolgt, welche Industriekamera (7) die dem mittigen Prüfstempel (5) gegenüberliegende beschichtete Seite des Biegebalkens (1) erfasst; wobei

- die Prüfvorrichtung derart konfiguriert ist, dass der Drei-Punkt-Biegeversuch endet, sobald die Industriekamera (7) in einer Rissbildungszone (R) der beschichteten Seite des Biegebalkens (1) eine Rissbildung mit Haarrissen im Wandanstrich (20) detektiert.

13. Prüfaufbau nach Anspruch 11 oder 12, **dadurch gekennzeichnet, dass** nach Beendigung des Drei-Punkt-Biegeversuchs die Mess-Software der Prüfvorrichtung die Längenänderung $\Delta_L$ des am Biegebalken (1) aufgetragenen Wandanstrichs (20) oder Wandbeschichtungssystems (25) als Differenz zumindest einer Zustandslänge ($L_1$) bei Rissbildung, welche Zustandslänge ($L_1$) dem Abstandsmaß zwischen Messpunkten (M) des zumindest einen Probenlängenintervalls ($L_{R1}$, $L_{R2}$, $L_{R3}$) bei Rissbildung am deformierten Biegebalken (1) entspricht, und der Ausgangslänge ($L_0$) des zumindest einen Probenlängenintervalls ($L_{P1}$, $L_{P2}$, $L_{P3}$) am unverformten Biegebalken (1) durch Berechnung ermittelt.

**Fig. 1**

**Fig. 1A**

**Fig. 1B**

**Fig. 2**

**Fig. 2A**

**Fig. 2B**

**Fig. 3**

**Fig. 4**

Fig. 5

Fig. 5A

**Fig. 6**

**Fig. 6A**

**Europäisches Patentamt**
**European Patent Office**
**Office européen des brevets**

# EUROPÄISCHER RECHERCHENBERICHT

Nummer der Anmeldung

EP 19 17 7904

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | IWAI TAKATSUGU: "Crack Bridging Properties of Elastomeric Waterproofing Wall Coatings", KAJIMA INSTITUTE OF CONSTRUCTION TECHNOLOGY, 2. August 2000 (2000-08-02), XP055601140, * Seite 396, Spalte 2 * * Seite 396, Spalte 1 * * Tabelle 1 * * Abbildung 1 * * Tabelle 2 * ----- | 1-13 | INV. G01B11/16 G01N3/20 ADD. G01N33/32 |
| A | Don Instron: "AVE 2 Non-Contacting Video Extensometer", , 1. Januar 2015 (2015-01-01), XP055638198, Gefunden im Internet: URL:https://www.instron.us/-/media/literature-library/products/2014/12/ave-2--non-contacting-video-extensometer.pdf [gefunden am 2019-11-01] * Seite 3 * ----- | 1-13 | |
| A | John Fletcher ET AL: "Paint and Coating Testing Manuel - 15th Edition of the Gardner-Sward Handbook", , 31. Januar 2012 (2012-01-31), XP055638101, Gefunden im Internet: URL:https://compass.astm.org/DIGITAL_LIBRARY/MNL/SOURCE_PAGES/MNL17-2ND.htm [gefunden am 2019-10-31] * Seite 638 * * Seite 751 * ----- | 1-13 | RECHERCHIERTE SACHGEBIETE (IPC) G01N G01B |

Der vorliegende Recherchenbericht wurde für alle Patentansprüche erstellt

| Recherchenort | Abschlußdatum der Recherche | Prüfer |
|---|---|---|
| Den Haag | 11. November 2019 | Mensink, Rob |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

.....................................................................
& : Mitglied der gleichen Patentfamilie, übereinstimmendes Dokument

EPO FORM 1503 03.82 (P04C03)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19802716 C2
- DD 266175 A1